(19) Europäisches Patentamt
European Patent Office
Office européen des brevets

(11) **EP 4 763 988 A1**

(12) **EUROPEAN PATENT APPLICATION**
published in accordance with Art. 153(4) EPC


(43) Date of publication:
**24.06.2026 Bulletin 2026/26**

(21) Application number: **24853799.5**

(22) Date of filing: **14.08.2024**

(51) International Patent Classification (IPC):
***C12N 15/13*** *(2006.01)* ***A61K 39/395*** *(2006.01)*
***A61P 35/00*** *(2006.01)* ***C07K 16/28*** *(2006.01)*

(52) Cooperative Patent Classification (CPC):
**A61K 39/395; A61P 35/00; C07K 16/00; C07K 16/28**

(86) International application number:
**PCT/CN2024/111997**

(87) International publication number:
**WO 2025/036392 (20.02.2025 Gazette 2025/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.08.2023 CN 202311027138**



(71) Applicants:
- **Minghui Pharmaceutical (Hangzhou) Ltd.
Hangzhou, Zhejiang 310018 (CN)**
- **Minghui Pharmaceutical (Shanghai) Limited
Shanghai 201210 (CN)**

(72) Inventors:
- **LIU, Bo
Hangzhou, Zhejiang 310018 (CN)**
- **ZHU, Liyuan
Shanghai 201210 (CN)**
- **CAO, Guoqing
Shanghai 201210 (CN)**
- **SHI, Junwei
Shanghai 201210 (CN)**
- **WENG, Xialian
Hangzhou, Zhejiang 310018 (CN)**
- **CAO, Yuting
Hangzhou, Zhejiang 310018 (CN)**

(74) Representative: **SONN Patentanwälte GmbH & Co KG
Riemergasse 14
1010 Wien (AT)**


Remarks:
The complete document including Reference Table(s) and the Sequence Listing(s) can be downloaded from the EPO website

(54) **SINGLE-DOMAIN ANTIBODY SPECIFICALLY BINDING TO CDCP1 AND USE THEREOF**


(57) A single-domain antibody specifically binding to CDCP 1 or an antigen-binding fragment thereof, a polypeptide construct and a conjugate containing the single-domain antibody or the antigen-binding fragment thereof, a nucleic acid molecule encoding the single-domain antibody or the antigen-binding fragment thereof and a host cell containing same, and a related use.


Killing curves of MHAB10-16 humanized antibody-Fab-ZAP in HCT116 cells

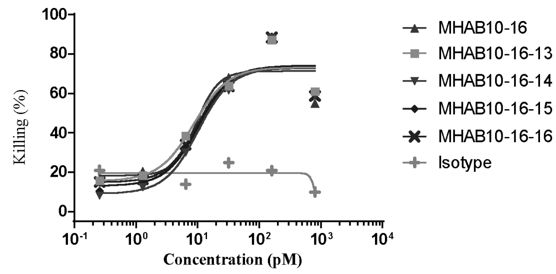


Fig. 14

EP 4 763 988 A1

## Description

### Technical Field

The present invention relates to a single-domain antibody or antigen-binding fragment thereof specifically binding to CDCP1, a polypeptide construct and conjugate comprising the single-domain antibody or antigen-binding fragment thereof, a nucleic acid molecule encoding the single-domain antibody or antigen-binding fragment thereof and a host cell containing the same, and related uses thereof.

### Background

CUB-domain containing protein 1 (CDCP1) is a type I transmembrane protein consisting of 836 amino acids in total length. CDCP1 encodes a 135 kDa protein with three CUB domains in the extracellular region. The amino acid sites at positions 368/369 or 369/370 between CUB1 domain and CUB2 domain are hydrolyzed and cleaved by serine protease to produce a 70 kDa fragment (cleaved CDCP1). Overexpression and hydrolysis activation of CDCP1 contribute to the loss of cell adhesion, increased migration and poor prognosis/survival in tumor patients. There are 14 N-glycosylation sites on the N-terminal side of the extracellular domain. It has been observed that N-glycosylation is a prerequisite for CDCP1 protein stability and plasma membrane localization, and the degree of glycosylation is correlated with the metastasis of cancer cells. The cytoplasmic domain of CDCP1 contains 5 conserved tyrosine residues, which are phosphorylation sites of Src family kinases (SFKs, such as Src, Fyn and Yes).

CDCP1 is highly expressed in tumor cells such as colorectal cancer, renal cancer, lung cancer, pancreatic cancer, breast cancer, and ovarian cancer, and increases the migration and invasiveness of cancer cells and cell survival rate by interacting with important signal transduction pathways in tumor invasiveness. Overexpression of CDCP1 is associated with poor tumor prognosis.

Patent US20080008719A1 describes an anti-hCDCP1 monoclonal antibody (clone name: 25A11) and a screening method, a diagnostic method, and a therapeutic method for prostate cancer using the antibody, and discloses that an ADC (antibody-drug-conjugate) that 25A11 antibody conjugated with saporin exhibits cytotoxic activity against PC-3 cancer cell lines in vitro, and exerts significant tumor growth inhibition activity by intravenous administration of the antibody in the form of ADC. Patent US20200181281A1 describes a plurality of anti-CDCP1 antibodies, and discloses that the anti-hCDCP1 antibodies in the form of ADC obtained using MMAE (monomethyl auristatin E) exhibits cytotoxic activity against human colon cancer cell lines or breast cancer cell lines in vitro, and exhibits anti-tumor activity in a mouse transplanted tumor model into which a human colon cancer cell line or breast cancer cell line is transplanted. Patent CN115176013A describes a plurality of anti-CDCP1 antibodies capable of binding to human CDCP1 and having low binding to human CD34-positive cells, and discloses that the anti-hCDCP1 antibody in the form of ADC obtained using pyrrolobenzodiazepine (PBD) exhibits cytotoxic activity against different tumor cell lines in vitro, and shows anti-tumor activity in mouse transplanted tumor model into which a human colon cancer cell line or prostate cancer cell line is transplanted.

The molecular weight of conventional antibodies is about 150 kDa. The molecular weight of single-domain antibodies or nanobodies derived from alpacas and fused with human Fc is only about 75kDa, which is only half of that of conventional antibodies, so that they can penetrate into the core part of tumors, maximize the chance of antibody drugs binding to tumor cells, and produce better diagnosis and treatment of tumors.

Therefore, providing a single-domain antibody targeting CDCP1 will have a better prospect for diagnosis and treatment of tumors.

### Contents of the present invention

The inventors of the present application have screened and obtained a series of single-domain antibodies and polypeptide constructs targeting CDCP1 after extensive research. The single-domain antibodies or polypeptide constructs have high binding activity to CDCP1. In particular, the single-domain antibodies or polypeptide constructs of the present invention can increase the endocytic activity of tumor cells and have strong killing activity against tumor cells. On this basis, the humanized single-domain antibodies or polypeptide constructs prepared from the single-domain antibodies still have these outstanding activities. In addition, the single-domain antibodies or polypeptide constructs also have the characteristics of small molecular weight and easy production.

Based on this, the present application also provides a conjugate comprising the single-domain antibody or antigen-binding fragment thereof, a nucleic acid molecule encoding the single-domain antibody or antigen-binding fragment thereof and a host cell comprising the same, as well as related uses thereof.

single-domain antibody and antigen-binding fragment thereof

Therefore, in one aspect, the present application provides a single-domain antibody or antigen-binding fragment thereof capable of specifically binding to CUB domain-containing protein 1 (CDCP1), wherein the single-domain antibody or antigen-binding fragment thereof comprises:

(a) a CDR1, which has: a sequence as set forth in any one of SEQ ID NOs: 1, 4, 7, or a sequence having a substitution, deletion or addition of one or more amino acids (e.g., a substitution, deletion or addition of 1, 2 or 3 amino acids) as compared to the sequence as set forth in any one of SEQ ID NOs: 1, 4, 7;

(b) a CDR2, which has: a sequence as set forth in any one of SEQ ID NOs: 2, 5, 8, or a sequence having a substitution, deletion or addition of one or more amino acids (e.g., a substitution, deletion or addition of 1, 2 or 3 amino acids) as compared to the sequence as set forth in any one of SEQ ID NOs: 2, 5, 8; and

(c) a CDR3, which has: a sequence as set forth in any one of SEQ ID NOs: 3, 6, 9, or a sequence having a substitution, deletion or addition of one or more amino acids (e.g., a substitution, deletion or addition of 1, 2 or 3 amino acids) as compared to the sequence as set forth in any one of SEQ ID NOs: 3, 6, 9.

In certain embodiments, the substitution is a conservative substitution.

In certain embodiments, the single-domain antibody or antigen-binding fragment thereof comprises: a CDR1 as set forth in SEQ ID NO: 1; a CDR2 as set forth in SEQ ID NO: 2; and, a CDR3 as set forth in SEQ ID NO: 3.

In certain embodiments, the single-domain antibody or antigen-binding fragment thereof comprises: a CDR1 as set forth in SEQ ID NO: 4; a CDR2 as set forth in SEQ ID NO: 5; and, a CDR3 as set forth in SEQ ID NO: 6.

In certain embodiments, the single-domain antibody or antigen-binding fragment thereof comprises: a CDR1 as set forth in SEQ ID NO: 7; a CDR2 as set forth in SEQ ID NO: 8; and, a CDR3 as set forth in SEQ ID NO: 9.

On the other hand, the present application provides a single-domain antibody or antigen-binding fragment thereof capable of specifically binding to CUB domain-containing protein 1 (CDCP1), wherein the single-domain antibody or antigen-binding fragment thereof comprises: 3 CDRs contained in the heavy chain variable region (VHH) as set forth in any one of SEQ ID NOs: 10-27.

In certain embodiments, the 3 CDRs contained in the VHH are defined by the Kabat, IMGT or Chothia numbering system.

In certain embodiments, the single-domain antibody or antigen-binding fragment thereof as described above comprises an amino acid sequence selected from the following:

(i) a sequence as set forth in any one of SEQ ID NOs: 10-27;

(ii) a sequence having a substitution, deletion or addition of one or more amino acids (e.g., a substitution, deletion or addition of 1, 2, 3, 4, 5, 6, 7 or 8 amino acids) as compared to the sequence as set forth in any one of SEQ ID NOs: 10-27; or

(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in any one of SEQ ID NOs: 10-27.

In certain embodiments, the substitution is a conservative substitution.

Single-domain antibody and antigen-binding fragment thereof

On the other hand, the present application provides a polypeptide construct capable of specifically binding to CDCP1, which comprises the single-domain antibody or antigen-binding fragment thereof as described above, and an immunoglobulin Fc domain.

In certain embodiments, the immunoglobulin Fc domain is connected to the N-terminal and/or C-terminal (e.g., C-terminal) of the single-domain antibody or antigen-binding fragment thereof optionally via a peptide linker.

In certain embodiments, the immunoglobulin Fc domain is an Fc domain of IgG, such as an IgG1 IgG2, IgG3 or IgG4 heavy chain constant region.

In certain embodiments, the immunoglobulin Fc domain comprises a sequence as set forth in SEQ ID NO: 28 or 29, or a sequence having a substitution, deletion or addition of one or more amino acids or any combination thereof (e.g., a sequence having a substitution, deletion or addition of up to 20, up to 15, up to 10, or up to 5 amino acids or any combination

thereof; for example, a sequence having a substitution, deletion or addition of 1, 2, 3, 4 or 5 amino acids or any combination thereof) as compared thereto.

In certain embodiments, the polypeptide construct has a sequence as set forth in any one of SEQ ID NOs: 30-47.

Isolated nucleic acid molecule

On the other hand, the present application also provides an isolated nucleic acid molecule, which encodes the single-domain antibody or antigen-binding fragment thereof as described above, or the polypeptide construct as described above.

Vector

On the other hand, the present application also provides a vector, which comprises the nucleic acid molecule as described above. In certain embodiments, the vector is a cloning vector or an expression vector.

Host cell

On the other hand, the present application also provides a host cell, which comprises the nucleic acid molecule or the vector as described above. Such host cells include, but are not limited to, prokaryotic cells such as bacterial cells (e.g., *Escherichia coli* cells), and eukaryotic cells such as fungal cells (e.g., yeast cells), insect cells, plant cells and animal cells (e.g., mammalian cells, such as mouse cells, human cells, etc.). In certain embodiments, the host cell is a microorganism.

Preparation method

The single-domain antibody of the present invention can be prepared by various methods known in the art, such as by genetic engineering recombinant technology. For example, a DNA molecule encoding the single-domain antibody of the present invention is obtained by chemical synthesis or PCR amplification. The obtained DNA molecule is inserted into an expression vector and then transformed/transfected into a host cell. Then, the transformed/transfected host cell is cultured under a specific condition and the single-domain antibody of the present invention is expressed.

The antigen-binding fragment of the present invention can be obtained by hydrolyzing the intact nanobody molecule (see, Morimoto et al., J. Biochem. Biophys. Methods 24:107-117 (1992) and Brennan et al., Science 229:81 (1985)). In addition, these antigen-binding fragments can also be produced directly by recombinant host cells (reviewed in Hudson, Curr. Opin. Immunol. 11: 548-557 (1999); Little et al., Immunol. Today, 21: 364-370 (2000)). Ordinary technicians in this field are fully aware of other techniques for preparing these antigen-binding fragments.

On the other hand, the present application also provides a method for preparing the single-domain antibody or antigen-binding fragment thereof as described above or the polypeptide construct as described above, which comprises under a condition that allows protein expression, culturing the host cell as described above, and recovering the single-domain antibody or antigen-binding fragment thereof or the polypeptide construct as described above from a culture of the cultured host cell.

Bispecific or multispecific antibody

On the other hand, the present application also provides a bispecific or multispecific antibody, which comprises the single-domain antibody or antigen-binding fragment thereof as described above or the polypeptide construct as described above.

In certain embodiments, the bispecific or multispecific antibody is capable of specifically binding to CDCP1, and additionally specifically binding to one or more other targets.

In certain embodiments, the bispecific or multispecific antibody further comprises at least one second antibody having a second binding specificity for a second target.

Conjugate

On the one hand, the present application also provides a conjugate, which comprises the single-domain antibody or antigen-binding fragment thereof as described above or the polypeptide construct as described above, and a therapeutic agent connected to the single-domain antibody or antigen-binding fragment thereof.

In certain embodiments, the therapeutic agent is selected from cytotoxic agents.

In certain embodiments, the therapeutic agent is selected from alkylating agent, mitotic inhibitor, antitumor antibiotic, antimetabolite, topoisomerase inhibitor, tyrosine kinase inhibitor, radionuclide agent, and any combination

thereof.

In certain embodiments, the conjugate is an antibody-drug conjugate (ADC).

Pharmaceutical composition

In one aspect, the present application also provides a pharmaceutical composition, which comprises the single-domain antibody or antigen-binding fragment thereof as described above, or the polypeptide construct as described above, or the bispecific or multispecific antibody as described above, or the conjugate as described above, and a pharmaceutically acceptable carrier and/or excipient.

In certain embodiments, the pharmaceutical composition further comprises an additional pharmaceutically active agent.

In certain embodiments, the additional pharmaceutically active agent is a drug with anti-tumor activity, such as an alkylating agent, a mitotic inhibitor, an antitumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor, or an oncolytic virus.

In certain embodiments, the single-domain antibody or antigen-binding fragment thereof, the bispecific or multispecific antibody or the conjugate and the additional pharmaceutically active agent are provided as separate components or as components of the same composition.

Kit

On the other hand, the present application also provides a kit, which comprises a single-domain antibody or antigen-binding fragment thereof as described above or the polypeptide construct as described above.

In certain embodiments, the single-domain antibody or antigen-binding fragment thereof or the polypeptide construct carries a detectable label, such as an enzyme (e.g., horseradish peroxidase), a radionuclide, a fluorescent dye, a luminescent substance (e.g., a chemiluminescent substance) or biotin.

In certain embodiments, the kit further comprises a second antibody, which specifically recognizing the single-domain antibody or antigen-binding fragment thereof as described above or the polypeptide construct as described above.

In certain embodiments, the second antibody further comprises a detectable label, such as an enzyme (e.g., horseradish peroxidase), a radionuclide, a fluorescent dye, a luminescent substance (e.g., a chemiluminescent substance) or biotin.

Chimeric antigen receptor

On the other hand, the present application also provides a chimeric antigen receptor, which comprises an antigen-binding domain of the single-domain antibody or antigen-binding fragment thereof as described above or the polypeptide construct as described above.

In certain embodiments, the antigen-binding domain is expressed by an immune effector cell (e.g., a T cell).

Isolated nucleic acid molecule

On the other hand, the present application also provides an isolated nucleic acid molecule, which encodes the chimeric antigen receptor as described above.

Vector

On the other hand, the present application also provides a vector, which comprises the isolated nucleic acid molecule as described above. In certain embodiments, it is used to prepare a chimeric antigen receptor T cell.

Host cell

On the other hand, the present application also provides a host cell, which comprises the isolated nucleic acid molecule as described above or the vector as described above. In certain embodiments, the host cell is an immune effector cell (e.g., a T cell or a NK cell). In certain embodiments, the host cell is a chimeric antigen receptor T cell (CAR-T).

Uses

On the other hand, the present application also provides a use of the single-domain antibody or antigen-binding

fragment thereof as described above, or the polypeptide construct as described above, or the bispecific or multispecific antibody as described above, or the conjugate as described above, or the pharmaceutical composition as described above, or the kit as described above, or the chimeric antigen receptor as described above, in the manufacture of a medicament for preventing and/or treating a tumor in a subject.

In certain embodiments, the tumor expresses CDCP1.

In certain embodiments, the medicament is used for inhibiting the growth of a tumor cell expressing CDCP1 and/or killing the tumor cell.

In certain embodiments, the medicament further comprises an additional pharmaceutically active agent.

In certain embodiments, the additional pharmaceutically active agent is a drug with anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor or an oncolytic virus.

In certain embodiments, the tumor is selected from the group consisting of non-small cell lung cancer, small cell lung cancer, renal cell carcinoma, colorectal cancer, ovarian cancer, breast cancer, pancreatic cancer, breast cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin lymphoma (CHL), primary mediastinal large B-cell lymphoma, B-cell rich lymphoma of T-cell/histiocyte, EBV-positive and negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin lymphoma, central nervous system (CNS) tumors, such as primary CNS lymphoma, spinal axis tumor, brainstem glioma.

In some embodiments, the subject is a mammal, such as a human.

On the other hand, the present application also provides a use of the single-domain antibody or antigen-binding fragment thereof as described above or the polypeptide construct as described above in the manufacture of a kit, in which the kit is used for detecting whether a tumor can be treated by an anti-tumor therapy targeting CDCP1.

In some embodiments, the antibody or antigen-binding fragment thereof carries a detectable label.

In certain embodiments, the tumor is selected from the group consisting of non-small cell lung cancer, small cell lung cancer, renal cell carcinoma, colorectal cancer, ovarian cancer, breast cancer, pancreatic cancer, breast cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin lymphoma (CHL), primary mediastinal large B-cell lymphoma, B-cell-rich lymphoma of T-cell/histiocyte, EBV-positive and negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin lymphoma, central nervous system (CNS) tumors, such as primary CNS lymphoma, spinal axis tumor, brainstem glioma.

Method

On the other hand, the present application also provides a method for detecting the presence or amount of CDCP1 (e.g., human CDCP1) in a sample, comprising the following steps:

(1) contacting the sample with the single-domain antibody or antigen-binding fragment thereof as described above or the polypeptide construct as described above;

(2) detecting the formation of a complex between the antibody or antigen-binding fragment thereof or the polypeptide construct and CDCP1 or detecting the amount of the complex.

In certain embodiments, the antibody or antigen-binding fragment thereof or the polypeptide construct carries a detectable label.

In certain embodiments, the method is used for therapeutic purposes, diagnostic purposes, or non-therapeutic non-diagnostic purposes.

On the other hand, the present application also provides a method for reducing an expression level of CDCP1 on the surface of a cell, which comprises contacting the cell with the single-domain antibody or antigen-binding fragment thereof as described above, or the polypeptide construct as described above, or the bispecific or multispecific antibody as described above, or the conjugate as described above, or the pharmaceutical composition as described above, or the kit as described above, so that the expression level of CDCP1 on the surface of the cell is reduced; wherein, the cell expresses CDCP1 on its surface.

In certain embodiments, the cell is a tumor cell expressing CDCP1.

In certain embodiments, the method is used for therapeutic purposes, diagnostic purposes, or non-therapeutic non-diagnostic purposes.

On the other hand, the present application also provides a method for preventing and/or treating a tumor, the method comprising administering an effective amount of the single-domain antibody or antigen-binding fragment thereof as described above, or the polypeptide construct as described above, or the bispecific or multispecific antibody as described above, or the conjugate as described above, or the pharmaceutical composition as described above, or the kit as described above, or the chimeric antigen receptor as described above to a subject in need thereof.

In certain embodiments, the tumor expresses CDCP1.

In certain embodiments, the tumor is selected from the group consisting of non-small cell lung cancer, small cell lung cancer, renal cell carcinoma, colorectal cancer, ovarian cancer, breast cancer, pancreatic cancer, breast cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin lymphoma (CHL), primary mediastinal large B-cell lymphoma, B-cell-rich lymphoma of T-cell/histiocyte, EBV-positive and negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin lymphoma, central nervous system (CNS) tumors, such as primary CNS lymphoma, spinal axis tumor, brainstem glioma.

In some embodiments, the subject is a mammal, such as a human.

On the other hand, the present application also provides a method for detecting whether a tumor can be treated by an anti-tumor therapy targeting CDCP1, the method comprising administering an effective amount of the single-domain antibody or antigen-binding fragment thereof as described above or the polypeptide construct as described above to a subject in need thereof.

In some embodiments, the single-domain antibody or antigen-binding fragment thereof carries a detectable label.

In certain embodiments, the tumor is selected from the group consisting of non-small cell lung cancer, small cell lung cancer, renal cell carcinoma, colorectal cancer, ovarian cancer, breast cancer, pancreatic cancer, breast cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin lymphoma (CHL), primary mediastinal large B-cell lymphoma, B-cell-rich lymphoma of T-cell/histiocyte, EBV-positive and negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin lymphoma, central nervous system (CNS) tumors, such as primary CNS lymphoma, spinal axis tumor, brainstem glioma.

## Definition of terms

In the present invention, unless otherwise specified, the scientific and technical terms used herein have the meanings commonly understood by those skilled in the art. In addition, the virology, biochemistry, and immunology laboratory operation steps used herein are all routine steps widely used in the corresponding fields. At the same time, in order to better understand the present invention, the definitions and explanations of relevant terms are provided below.

When the terms "for example", "such as", "e.g.", "include", "comprise" or their variations are used herein, these terms will not be considered as restrictive terms, but will be interpreted as meaning "but not limited to" or "not limited to".

Unless otherwise specified herein or clearly contradicted by the context, the terms "a" and "an" and "the" and similar referents should be interpreted as covering the singular and plural in the context of describing the present invention (especially in the context of the following claims).

As used herein, the term "single-domain antibody" or "nanobody" has the meaning commonly understood by those skilled in the art, which refers to an antibody fragment composed of a single monomer variable antibody domain (e.g., a single heavy chain variable region), which is usually derived from a variable region of a heavy chain antibody (e.g., a camelid antibody or a shark antibody). Typically, a nanobody consists of 4 framework regions and 3 complementarity determining regions, and has a structure of FR1-CDR1-FR2-CDR2-FR3-CDR3-FR4. A nanobody can be truncated at the N-terminal or C-terminal so that it contains only part of FR1 and/or FR4, or lacks one or two of those framework regions, as long as it substantially maintains antigen-binding ability and specificity. Single-domain antibodies are also called nanobodies, and the two terms can be used interchangeably.

As used herein, the term "antigen-binding fragment" of single-domain antibody refers to a polypeptide comprising a fragment of single-domain antibody that retains the ability to specifically bind to the same antigen bound by the single-domain antibody, and/or competes with the single-domain antibody for specific binding to the antigen, which is also referred to as an "antigen-binding portion". See generally, Fundamental Immunology, Ch. 7 (Paul, W., ed., 2nd edition, Raven Press, N.Y. (1989), which is incorporated herein by reference in its entirety for all purposes. The antigen-binding

fragment of the antibody of the present invention can be produced by recombinant DNA technology or by enzymatic or chemical cleavage of the nanobody of the present invention. In some embodiments, the "antigen-binding fragment" of single-domain antibody can be truncated at the N-terminal or C-terminal as compared to the full-length single-domain antibody so that it contains only part of FR1 and/or FR4, or lacks one or two of those framework regions, as long as it substantially maintains antigen-binding ability and specificity.

The antigen-binding fragment of single-domain antibody can be obtained from a given single-domain antibody (e.g., the nanobody provided by the present invention) using conventional techniques known to those skilled in the art (e.g., recombinant DNA technology or enzymatic or chemical cleavage method), and the antigen-binding fragment of single-domain antibody is are screened for specificity in the same manner as for the intact nanobody.

As used herein, unless the context clearly indicates, when the term "single-domain antibody" is mentioned, it includes not only the intact single-domain antibody, but also an antigen-binding fragment of the single-domain antibody.

As used herein, the term "complementarity determining region" or "CDR" refers to amino acid residues in a variable region of an antibody that are responsible for antigen binding. There are three CDRs in a nanobody, named CDR1, CDR2 and CDR3. The precise boundaries of these CDRs can be defined according to various numbering systems known in the art, for example, according to the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al., Dev. Comparat. Immunol. 27:55-77, 2003). For a given nanobody, a person skilled in the art will easily identify the CDRs defined by each numbering system. Moreover, the correspondence between different numbering systems is well known to a person skilled in the art (see, for example, Lefranc et al., Dev. Comparat. Immunol. 27:55-77, 2003). In this context, the CDRs of nanobody are preferably determined by the Kabat, Chothia and/or IMGT numbering systems.

As used herein, the term "framework region" or "FR" residues refers to those amino acid residues in the variable region of an antibody other than the CDR residues as defined above.

As used herein, the term "identity" is used to refer to the matching of sequences between two polypeptides or between two nucleic acids. In order to determine the percent identity of two amino acid sequences or two nucleic acid sequences, the sequences are aligned for optimal comparison purposes (e.g., a gap may be introduced in the first amino acid sequence or nucleic acid sequence for optimal alignment with the second amino acid or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, the molecules are identical at that position. The percent identity between the two sequences is a function of the number of identical positions shared by the sequences (i.e., percent identity = number of identical overlapping positions/total number of positions $\times$ 100%). In certain embodiments, the two sequences are of the same length.

The determination of the percent identity between two sequences can also be achieved using a mathematical algorithm. A non-limiting example of mathematical algorithm used for the comparison of two sequences is the algorithm of Karlin and Altschul, 1990, Proc. Natl. Acad. Sci. U.S.A. 87:2264-2268, as modified by Karlin and Altschul, 1993, Proc. Natl. Acad. Sci. U.S.A. 90:5873-5877. Such algorithms are integrated into the NBLAST and XBLAST programs of Altschul et al., 1990, J. Mol. Biol. 215:403.

As used herein, the term "specific binding" refers to a non-random binding reaction between two molecules, such as a reaction between an antibody and an antigen to which it is directed. The strength or affinity of a specific binding interaction can be represented by the equilibrium dissociation constant ($K_D$) of the interaction. In the present invention, the term "$K_D$" refers to the dissociation equilibrium constant of a specific antibody-antigen interaction, which is used to describe the binding affinity between an antibody and an antigen. The smaller the equilibrium dissociation constant, the tighter the antibody-antigen binding and the higher the affinity between the antibody and the antigen.

The specific binding properties between two molecules can be determined using methods known in the art. One method involves measuring the speed of formation and dissociation of antigen binding site/antigen complex. Both "association rate constant" (ka or kon) and "dissociation rate constant" (kdis or koff) can be calculated by concentrations and the actual rates of association and dissociation (see Malmqvist M, Nature, 1993, 361: 186-187). The ratio of kdis/kon is equal to the dissociation constant $K_D$ (see Davies et al., Annual Rev Biochem, 1990; 59: 439-473). $K_D$, kon and kdis values can be measured by any effective method. In certain embodiments, surface plasmon resonance (SPR) can be used to measure the dissociation constant in Biacore. In addition, bioluminescence interferometry or Kinexa can be used to measure the dissociation constant.

As used herein, the detectable label of the present invention can be any substance that can be detected by fluorescence, spectroscopy, photochemistry, biochemistry, immunology, electricity, optical or chemical means. Such labels are well known in the art, and examples thereof include, but are not limited to, enzymes (e.g., horseradish peroxidase, alkaline phosphatase, $\beta$-galactosidase, urease, glucose oxidase, etc.), radionuclides (e.g., $^{3}H$, $^{125}I$, $^{35}S$, $^{14}C$ or $^{32}P$), fluorescent dyes (e.g., fluorescein isothiocyanate (FITC), fluorescein, tetramethylrhodamine isothiocyanate

(TRITC), phycoerythrin (PE), Texas Red, rhodamine, quantum dots or cyanine dye derivatives (e.g., Cy7, Alexa 750), luminescent substances (e.g., chemiluminescent substances, such as acridinium ester compounds, luminol and its derivatives, ruthenium derivatives such as terpyridine ruthenium), magnetic beads (e.g., Dynabeads®), calorimetric labels such as colloidal gold or colored glass or plastic (e.g., polystyrene, polypropylene, latex, etc.) beads, and biotin for binding to avidin (e.g., streptavidin) modified with the above labels.

As used herein, the term "vector" refers to a nucleic acid vehicle into which a polynucleotide can be inserted. When a vector can express the protein encoded by the inserted polynucleotide, the vector is called an expression vector. The vector can be introduced into a host cell by transformation, transduction or transfection so that the genetic material elements it carries are expressed in the host cell. Vectors are well known to those skilled in the art, and include but are not limited to: plasmids; phagemids; cosmids; artificial chromosomes, such as yeast artificial chromosome (YAC), bacterial artificial chromosome (BAC) or P1-derived artificial chromosome (PAC); bacteriophages such as λ phage or M13 phage, and animal viruses. Animal viruses that can be used as vectors include but are not limited to retrovirus (including lentivirus), adenovirus, adeno-associated virus, herpes virus (e.g., herpes simplex virus), poxvirus, baculovirus, papillomaviruses, papovavirus (e.g., SV40). A vector can contain a variety of elements that control expression, including but not limited to promoter sequence, transcription start sequence, enhancer sequence, selection element and reporter gene. In addition, the vector may also contain an origin of replication.

As used herein, the term "host cell" refers to a cell that can be used to introduce a vector, including but not limited to prokaryotic cell such as *Escherichia coli* or *Bacillus subtilis,* fungal cell such as yeast cell or *Aspergillus,* insect cell such as *S2 Drosophila* cell or Sf9, or animal cell such as fibroblast, CHO cell, COS cell, NSO cell, HeLa cell, BHK cell, HEK 293 cell or human cell.

As used herein, the term "conservative substitution" refers to an amino acid substitution that does not adversely affect or change the expected properties of a protein/polypeptide comprising the amino acid sequence. For example, conservative substitutions can be introduced by standard techniques known in the art, such as site-directed mutagenesis and PCR-mediated mutagenesis. Conservative amino acid substitutions include substitutions of amino acid residues with amino acid residues having similar side chains, such as substitutions with residues that are physically or functionally similar to the corresponding amino acid residues (e.g., having similar size, shape, charge, chemical properties, including the ability to form covalent bond or hydrogen bond, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). Thus, it is preferred to replace a corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al. Protein Eng. 12(10):879-884 (1999); and Burks et al. Proc. Natl Acad. Set USA 94:412-417 (1997), which are incorporated herein by reference).

The twenty conventional amino acids referred to herein are written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by single-letter and three-letter abbreviations known in the art. For example, alanine can be represented by A or Ala.

Beneficial effects of the present invention

The single-domain antibody or polypeptide construct targeting CDCP1 provided in the present application has high binding activity with CDCP1. In particular, the single-domain antibody or polypeptide construct of the present invention can increase the endocytic activity of tumor cells and has a strong killing activity against tumor cells. On this basis, the humanized single-domain antibody or polypeptide construct prepared by the single-domain antibody or polypeptide construct still has these outstanding activities. In addition, the single-domain antibody or polypeptide construct also has the characteristics of small molecular weight and easy production.

The embodiments of the present invention will be described in detail below in conjunction with the drawings and examples, but those skilled in the art will understand that the following drawings and examples are only used to illustrate the present invention, rather than to limit the scope of the present invention. According to the following detailed description of the drawings and preferred embodiments, the various objects and advantages of the present invention will become apparent to those skilled in the art.

**Brief Description of the Drawings**

Fig. 1 shows the binding activities of 18 nanobody constructs of the present application to human CDCP1 antigen detected by ELISA.

Fig. 2 shows the binding activities of 8 nanobody constructs of the present application to human CDCP1 antigen detected by ELISA.

Fig. 3 shows the binding activities of the nanobody constructs of the present application to HCT116 cells (Fig. 3A) and SW480 cells (Fig. 3B) in a FACS experiment.

Fig. 4 shows the binding activities of the nanobody constructs of the present application to PL45 cells in a FACS experiment.

Fig. 5 shows the results of endocytosis experiments of HCT116 cells (Fig. 5A) and SW480 cells (Fig. 5B).

Fig. 6 shows the killing activities of the nanobody constructs of the present application to HCT116 cells.

Fig. 7 A and Fig. 7B show the binding activities of the humanized nanobody constructs of the present application to human CDCP1 antigen detected by ELISA.

Fig. 8 shows the binding activities of the humanized nanobody constructs of the present application to HCT116 cells in a FACS experiment.

Fig. 9 shows the results of endocytosis experiments of HCT116 cells.

Fig. 10 shows the killing activities of the humanized nanobody constructs of the present application on HCT116 cells.

Fig. 11 shows the binding activities of the humanized nanobody constructs of the present application to human CDCP1 antigen detected by ELISA.

Fig. 12 shows the binding activities of the humanized nanobody constructs of the present application to HCT116 cells in a FACS experiment.

Fig. 13 shows the results of the endocytosis experiment of HCT116 cells.

Fig. 14 shows the killing activities of the humanized nanobody constructs of the present application on HCT116 cells.

Fig. 15 shows the binding activities of the humanized nanobody constructs of the present application to CDCP1 antigens of different species detected by ELISA.

Fig. 16 shows the binding activities of the humanized nanobody constructs of the present application to HCT116 cells in a FACS experiment.

Fig. 17 shows the binding activities of the humanized nanobody constructs of the present application to SW480 cells in a FACS experiment.

Fig. 18 shows the binding activities of the humanized nanobody constructs of the present application to PL45 cells in a FACS experiment.

Fig. 19 shows the binding activities of the humanized nanobody constructs of the present application to HT-29 cells in a FACS experiment.

Fig. 20 shows the binding activities of the humanized nanobody constructs of the present application to LNCaP cells in a FACS experiment.

Fig. 21 shows the killing activities of the humanized nanobody constructs of the present application to HCT116 cells.

Sequence information

The description of the sequences involved in the present application is provided in the table below.

Table 1: Sequence information

| SEQ ID NO: | Sequence name | Sequence |
|---|---|---|
| 1 | Kabat-CDR1 | ANGMV |
| 2 | Kabat-CDR2 | VISSSGSTNYADSVKG |
| 3 | Kabat-CDR3 | AHYGQTY |
| 4 | Chothia-CDR1 | GSDVSAN |
| 5 | Chothia-CDR2 | SSSGS |
| 6 | Chothia-CDR3 | AHYGQTY |
| 7 | IMGT-CDR1 | GSDVSANG |
| 8 | IMGT-CDR2 | ISSSGST |
| 9 | IMGT-CDR3 | GVAHYGQTY |
| 10 | MHAB10-16 VHH | QLQLVESGGGLVQPGGSLRLSCAASGSDVSANGMVWHRQAPGKQ REWVAVISSSGSTNYADSVKGRFTISRDSDKNTMYLQMNNLKPED TAVYLCGVAHYGQTYWGKGTLVTVSS |
| 11 | MHAB10-16-1 VHH | EVQLVESGGGLVQPGGSLRLSCAASGSDVSANGMVWHRQAPGKQ REWVAVISSSGSTNYADSVKGRFTISRDSDKNTMYLQMNSLRPED TAVYLCGVAHYGQTYWGKGTLVTVSS |
| 12 | MHAB10-16-2 VHH | EVQLVESGGGLVQPGGSLRLSCAASGSDVSANGMVWHRQAPGKG REWVAVISSSGSTNYADSVKGRFTISRDSDKNTMYLQMNSLRPED TAVYLCGVAHYGQTYWGKGTLVTVSS |
| 13 | MHAB10-16-3 VHH | EVQLVESGGGLVQPGGSLRLSCAASGSDVSANGMVWHRQAPGKG REWVAVISSSGSTNYADSVKGRFTISRDNSKNTMYLQMNSLRPED TAVYLCGVAHYGQTYWGKGTLVTVSS |
| 14 | MHAB10-16-4 VHH | EVQLVESGGGLVQPGGSLRLSCAASGSDVSANGMVWHRQAPGKG REWVAVISSSGSTNYADSVKGRFTISRDNSKNTMYLQMNSLRPED TAVYYCGVAHYGQTYWGKGTLVTVSS |
| 15 | MHAB10-16-5 VHH | EVQLVESGGGLVQPGGSLRLSCAASGSDVSANGMVWHRQAPGKG REWVAVISSSGSTNYADSVKGRFTISRDSDKNTMYLQMNSLRPED TAVYYCGVAHYGQTYWGKGTLVTVSS |
| 16 | MHAB10-16-6 VHH | EVQLVESGGGLVQPGGSLRLSCAASGSDVSANGMVWHRQAPGKQ REWVAVISSSGSTNYADSVKGRFTISRDNSKNTMYLQMNSLRPED TAVYLCGVAHYGQTYWGKGTLVTVSS |
| 17 | MHAB10-16-7 VHH | EVQLVESGGGLVQPGGSLRLSCAASGSDVSANGMVWHRQAPGKQ REWVAVISSSGSTNYADSVKGRFTISRDNSKNTMYLQMNSLRPED TAVYYCGVAHYGQTYWGKGTLVTVSS |
| 18 | MHAB10-16-8 VHH | EVQLVESGGGLVQPGGSLRLSCAASGSDVSANGMVWHRQAPGKQ REWVAVISSSGSTNYADSVKGRFTISRDSDKNTMYLQMNSLRPED TAVYYCGVAHYGQTYWGKGTLVTVSS |

(continued)

| SEQ ID NO: | Sequence name | Sequence |
|---|---|---|
| 19 | MHAB10-16-9 VHH | QLQLVESGGGLVQPGGSLRLSCAASGSDVSANGMVWHRQAPGKQ REWVAVISSSGSTNYADSVKGRFTISRDSDKNTMYLQMNSLRPED TAVYLCGVAHYGQTYWGKGTLVTVSS |
| 20 | MHAB10-16-10 VHH | EVQLVESGGGLVQPGGSLRLSCAASGSDVSANGMVWHRQAPGKQ REWVAVISSSGSTNYADSVKGRFTISRDSDKNTMYLQMNNLKPED TAVYLCGVAHYGQTYWGKGTLVTVSS |
| 21 | MHAB10-16-11 VHH | QLQLVESGGGLVQPGGSLRLSCAASGSDVSANGMVWHRQAPGKG REWVAVISSSGSTNYADSVKGRFTISRDSDKNTMYLQMNSLRPED TAVYLCGVAHYGQTYWGKGTLVTVSS |
| 22 | MHAB10-16-12 VHH | QLQLVESGGGLVQPGGSLRLSCAASGSDVSANGMVWHRQAPGKG REWVAVISSSGSTNYADSVKGRFTISRDNSKNTMYLQMNSLRPED TAVYLCGVAHYGQTYWGKGTLVTVSS |
| 23 | MHAB10-16-13 VHH | QLQLVESGGGLVQPGGSLRLSCAASGSDVSANGMVWHRQAPGKG REWVAVISSSGSTNYADSVKGRFTISRDNSKNTMYLQMNSLRPED TAVYYCGVAHYGQTYWGKGTLVTVSS |
| 24 | MHAB10-16-14 VHH | QLQLVESGGGLVQPGGSLRLSCAASGSDVSANGMVWHRQAPGKG REWVAVISSSGSTNYADSVKGRFTISRDSDKNTMYLQMNSLRPED TAVYYCGVAHYGQTYWGKGTLVTVSS |
| 25 | MHAB10-16-15 VHH | QLQLVESGGGLVQPGGSLRLSCAASGSDVSANGMVWHRQAPGKQ REWVAVISSSGSTNYADSVKGRFTISRDNSKNTMYLQMNSLRPED TAVYLCGVAHYGQTYWGKGTLVTVSS |
| 26 | MHAB10-16-16 VHH | QLQLVESGGGLVQPGGSLRLSCAASGSDVSANGMVWHRQAPGKQ REWVAVISSSGSTNYADSVKGRFTISRDNSKNTMYLQMNSLRPED TAVYYCGVAHYGQTYWGKGTLVTVSS |
| 27 | MHAB10-16-17 VHH | QLQLVESGGGLVQPGGSLRLSCAASGSDVSANGMVWHRQAPGKQ REWVAVISSSGSTNYADSVKGRFTISRDSDKNTMYLQMNSLRPED TAVYYCGVAHYGQTYWGKGTLVTVSS |
| 28 | Fc-1 | EPKSCDKTHTCPPCPAPEAAGGPSVFLFPPKPKDTLMISRTPEVTCV VVDVSHEDPEVKFNWYVDGVEVHNAKTKPREEQYNSTYRVVSVL TVLHQDWLNGKEYKCKVSNKALPAPIEKTISKAKGQPREPQVYTL PPSREEMTKNQVSLTCLVKGFYPSDIAVEWESNGQPENNYKTTPP VLDSDGSFFLYSKLTVDKSRWQQGNVFSCSVMHEALHNHYTQKS LSLSPGK |
| 29 | Fc-2 | EPKSSDKTHTCPPCPAPEAAGGPSVFLFPPKPKDTLMISRTPEVTCV VVDVSHEDPEVKFNWYVDGVEVHNAKTKPREEQYNSTYRVVSVL TVLHQDWLNGKEYKCKVSNKALPAPIEKTISKAKGQPREPQVYTL PPSREEMTKNQVSLTCLVKGFYPSDIAVEWESNGQPENNYKTTPP VLDSDGSFFLYSKLTVDKSRWQQGNVFSCSVMHEALHNHYTQKS LSLSPGK |

(continued)

| SEQ ID NO: | Sequence name | Sequence |
|---|---|---|
| 30 | MHAB10-16 | QLQLVESGGGLVQPGGSLRLSCAASGSDVSANGMVWHRQAPGKQ REWVAVISSSGSTNYADSVKGRFTISRDSDKNTMYLQMNNLKPED TAVYLCGVAHYGQTYWGKGTLVTVSSEPKSCDKTHTCPPCPAPE AAGGPSVFLFPPKPKDTLMISRTPEVTCVVVDVSHEDPEVKFNWY VDGVEVHNAKTKPREEQYNSTYRVVSVLTVLHQDWLNGKEYKC KVSNKALPAPIEKTISKAKGQPREPQVYTLPPSREEMTKNQVSLTC LVKGFYPSDIAVEWESNGQPENNYKTTPPVLDSDGSFFLYSKLTVD KSRWQQGNVFSCSVMHEALHNHYTQKSLSLSPGK |
| 31 | MHAB10-16-1 | EVQLVESGGGLVQPGGSLRLSCAASGSDVSANGMVWHRQAPGKQ REWVAVISSSGSTNYADSVKGRFTISRDSDKNTMYLQMNSLRPED TAVYLCGVAHYGQTYWGKGTLVTVSSEPKSSDKTHTCPPCPAPEA AGGPSVFLFPPKPKDTLMISRTPEVTCVVVDVSHEDPEVKFNWYV DGVEVHNAKTKPREEQYNSTYRVVSVLTVLHQDWLNGKEYKCK VSNKALPAPIEKTISKAKGQPREPQVYTLPPSREEMTKNQVSLTCL VKGFYPSDIAVEWESNGQPENNYKTTPPVLDSDGSFFLYSKLTVD KSRWQQGNVFSCSVMHEALHNHYTQKSLSLSPGK |
| 32 | MHAB10-16-2 | EVQLVESGGGLVQPGGSLRLSCAASGSDVSANGMVWHRQAPGKG REWVAVISSSGSTNYADSVKGRFTISRDSDKNTMYLQMNSLRPED TAVYLCGVAHYGQTYWGKGTLVTVSSEPKSSDKTHTCPPCPAPEA AGGPSVFLFPPKPKDTLMISRTPEVTCVVVDVSHEDPEVKFNWYV DGVEVHNAKTKPREEQYNSTYRVVSVLTVLHQDWLNGKEYKCK VSNKALPAPIEKTISKAKGQPREPQVYTLPPSREEMTKNQVSLTCL VKGFYPSDIAVEWESNGQPENNYKTTPPVLDSDGSFFLYSKLTVD KSRWQQGNVFSCSVMHEALHNHYTQKSLSLSPGK |
| 33 | MHAB10-16-3 | EVQLVESGGGLVQPGGSLRLSCAASGSDVSANGMVWHRQAPGKG REWVAVISSSGSTNYADSVKGRFTISRDNSKNTMYLQMNSLRPED TAVYLCGVAHYGQTYWGKGTLVTVSSEPKSSDKTHTCPPCPAPEA AGGPSVFLFPPKPKDTLMISRTPEVTCVVVDVSHEDPEVKFNWYV DGVEVHNAKTKPREEQYNSTYRVVSVLTVLHQDWLNGKEYKCK VSNKALPAPIEKTISKAKGQPREPQVYTLPPSREEMTKNQVSLTCL VKGFYPSDIAVEWESNGQPENNYKTTPPVLDSDGSFFLYSKLTVD KSRWQQGNVFSCSVMHEALHNHYTQKSLSLSPGK |
| 34 | MHAB10-16-4 | EVQLVESGGGLVQPGGSLRLSCAASGSDVSANGMVWHRQAPGKG REWVAVISSSGSTNYADSVKGRFTISRDNSKNTMYLQMNSLRPED TAVYYCGVAHYGQTYWGKGTLVTVSSEPKSSDKTHTCPPCPAPE AAGGPSVFLFPPKPKDTLMISRTPEVTCVVVDVSHEDPEVKFNWY VDGVEVHNAKTKPREEQYNSTYRVVSVLTVLHQDWLNGKEYKC KVSNKALPAPIEKTISKAKGQPREPQVYTLPPSREEMTKNQVSLTC LVKGFYPSDIAVEWESNGQPENNYKTTPPVLDSDGSFFLYSKLTVD KSRWQQGNVFSCSVMHEALHNHYTQKSLSLSPGK |
| 35 | MHAB10-16-5 | EVQLVESGGGLVQPGGSLRLSCAASGSDVSANGMVWHRQAPGKG REWVAVISSSGSTNYADSVKGRFTISRDSDKNTMYLQMNSLRPED TAVYYCGVAHYGQTYWGKGTLVTVSSEPKSSDKTHTCPPCPAPE AAGGPSVFLFPPKPKDTLMISRTPEVTCVVVDVSHEDPEVKFNWY VDGVEVHNAKTKPREEQYNSTYRVVSVLTVLHQDWLNGKEYKC KVSNKALPAPIEKTISKAKGQPREPQVYTLPPSREEMTKNQVSLTC LVKGFYPSDIAVEWESNGQPENNYKTTPPVLDSDGSFFLYSKLTVD KSRWQQGNVFSCSVMHEALHNHYTQKSLSLSPGK |

(continued)

| SEQ ID NO: | Sequence name | Sequence |
|---|---|---|
| 36 | MHAB10-16-6 | EVQLVESGGGLVQPGGSLRLSCAASGSDVSANGMVWHRQAPGKQREWVAVISSSGSTNYADSVKGRFTISRDNSKNTMYLQMNSLRPEDTAVYLCGVAHYGQTYWGKGTLVTVSSEPKSSDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 37 | MHAB10-16-7 | EVQLVESGGGLVQPGGSLRLSCAASGSDVSANGMVWHRQAPGKQREWVAVISSSGSTNYADSVKGRFTISRDNSKNTMYLQMNSLRPEDTAVYYCGVAHYGQTYWGKGTLVTVSSEPKSSDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 38 | MHAB10-16-8 | EVQLVESGGGLVQPGGSLRLSCAASGSDVSANGMVWHRQAPGKQREWVAVISSSGSTNYADSVKGRFTISRDSDKNTMYLQMNSLRPEDTAVYYCGVAHYGQTYWGKGTLVTVSSEPKSSDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 39 | MHAB10-16-9 | QLQLVESGGGLVQPGGSLRLSCAASGSDVSANGMVWHRQAPGKQREWVAVISSSGSTNYADSVKGRFTISRDSDKNTMYLQMNSLRPEDTAVYLCGVAHYGQTYWGKGTLVTVSSEPKSSDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 40 | MHAB10-16-10 | EVQLVESGGGLVQPGGSLRLSCAASGSDVSANGMVWHRQAPGKQREWVAVISSSGSTNYADSVKGRFTISRDSDKNTMYLQMNNLKPEDTAVYLCGVAHYGQTYWGKGTLVTVSSEPKSSDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| SEQ ID NO: | Sequence name | Sequence |
|---|---|---|
| 41 | MHAB10-16-11 | QLQLVESGGGLVQPGGSLRLSCAASGSDVSANGMVWHRQAPGKG REWVAVISSSGSTNYADSVKGRFTISRDSDKNTMYLQMNSLRPED TAVYLCGVAHYGQTYWGKGTLVTVSSEPKSSDKTHTCPPCPAPEA AGGPSVFLFPPKPKDTLMISRTPEVTCVVVDVSHEDPEVKFNWYV DGVEVHNAKTKPREEQYNSTYRVVSVLTVLHQDWLNGKEYKCK VSNKALPAPIEKTISKAKGQPREPQVYTLPPSREEMTKNQVSLTCL VKGFYPSDIAVEWESNGQPENNYKTTPPVLDSDGSFFLYSKLTVD KSRWQQGNVFSCSVMHEALHNHYTQKSLSLSPGK |
| 42 | MHAB10-16-12 | QLQLVESGGGLVQPGGSLRLSCAASGSDVSANGMVWHRQAPGKG REWVAVISSSGSTNYADSVKGRFTISRDNSKNTMYLQMNSLRPED TAVYLCGVAHYGQTYWGKGTLVTVSSEPKSSDKTHTCPPCPAPEA AGGPSVFLFPPKPKDTLMISRTPEVTCVVVDVSHEDPEVKFNWYV DGVEVHNAKTKPREEQYNSTYRVVSVLTVLHQDWLNGKEYKCK VSNKALPAPIEKTISKAKGQPREPQVYTLPPSREEMTKNQVSLTCL VKGFYPSDIAVEWESNGQPENNYKTTPPVLDSDGSFFLYSKLTVD KSRWQQGNVFSCSVMHEALHNHYTQKSLSLSPGK |
| 43 | MHAB10-16-13 | QLQLVESGGGLVQPGGSLRLSCAASGSDVSANGMVWHRQAPGKG REWVAVISSSGSTNYADSVKGRFTISRDNSKNTMYLQMNSLRPED TAVYYCGVAHYGQTYWGKGTLVTVSSEPKSSDKTHTCPPCPAPE AAGGPSVFLFPPKPKDTLMISRTPEVTCVVVDVSHEDPEVKFNWY VDGVEVHNAKTKPREEQYNSTYRVVSVLTVLHQDWLNGKEYKC KVSNKALPAPIEKTISKAKGQPREPQVYTLPPSREEMTKNQVSLTC LVKGFYPSDIAVEWESNGQPENNYKTTPPVLDSDGSFFLYSKLTVD KSRWQQGNVFSCSVMHEALHNHYTQKSLSLSPGK |
| 44 | MHAB10-16-14 | QLQLVESGGGLVQPGGSLRLSCAASGSDVSANGMVWHRQAPGKG REWVAVISSSGSTNYADSVKGRFTISRDSDKNTMYLQMNSLRPED TAVYYCGVAHYGQTYWGKGTLVTVSSEPKSSDKTHTCPPCPAPE AAGGPSVFLFPPKPKDTLMISRTPEVTCVVVDVSHEDPEVKFNWY VDGVEVHNAKTKPREEQYNSTYRVVSVLTVLHQDWLNGKEYKC KVSNKALPAPIEKTISKAKGQPREPQVYTLPPSREEMTKNQVSLTC LVKGFYPSDIAVEWESNGQPENNYKTTPPVLDSDGSFFLYSKLTVD KSRWQQGNVFSCSVMHEALHNHYTQKSLSLSPGK |
| 45 | MHAB10-16-15 | QLQLVESGGGLVQPGGSLRLSCAASGSDVSANGMVWHRQAPGKQ REWVAVISSSGSTNYADSVKGRFTISRDNSKNTMYLQMNSLRPED TAVYLCGVAHYGQTYWGKGTLVTVSSEPKSSDKTHTCPPCPAPEA AGGPSVFLFPPKPKDTLMISRTPEVTCVVVDVSHEDPEVKFNWYV DGVEVHNAKTKPREEQYNSTYRVVSVLTVLHQDWLNGKEYKCK VSNKALPAPIEKTISKAKGQPREPQVYTLPPSREEMTKNQVSLTCL VKGFYPSDIAVEWESNGQPENNYKTTPPVLDSDGSFFLYSKLTVD KSRWQQGNVFSCSVMHEALHNHYTQKSLSLSPGK |
| 46 | MHAB10-16-16 | QLQLVESGGGLVQPGGSLRLSCAASGSDVSANGMVWHRQAPGKQ REWVAVISSSGSTNYADSVKGRFTISRDNSKNTMYLQMNSLRPED TAVYYCGVAHYGQTYWGKGTLVTVSSEPKSSDKTHTCPPCPAPE AAGGPSVFLFPPKPKDTLMISRTPEVTCVVVDVSHEDPEVKFNWY VDGVEVHNAKTKPREEQYNSTYRVVSVLTVLHQDWLNGKEYKC KVSNKALPAPIEKTISKAKGQPREPQVYTLPPSREEMTKNQVSLTC LVKGFYPSDIAVEWESNGQPENNYKTTPPVLDSDGSFFLYSKLTVD KSRWQQGNVFSCSVMHEALHNHYTQKSLSLSPGK |

(continued)

| SEQ ID NO: | Sequence name | Sequence |
|---|---|---|
| 47 | MHAB10-16-17 | QLQLVESGGGLVQPGGSLRLSCAASGSDVSANGMVWHRQAPGKQ REWVAVISSSGSTNYADSVKGRFTISRDSDKNTMYLQMNSLRPED TAVYYCGVAHYGQTYWGKGTLVTVSSEPKSSDKTHTCPPCPAPE AAGGPSVFLFPPKPKDTLMISRTPEVTCVVVDVSHEDPEVKFNWY VDGVEVHNAKTKPREEQYNSTYRVVSVLTVLHQDWLNGKEYKC KVSNKALPAPIEKTISKAKGQPREPQVYTLPPSREEMTKNQVSLTC LVKGFYPSDIAVEWESNGQPENNYKTTPPVLDSDGSFFLYSKLTVD KSRWQQGNVFSCSVMHEALHNHYTQKSLSLSPGK |
| 48 | 25A11(MHAB10-PC1) HC | EVQLQQPGAELVKPGASVKMSCKASGYTFTSYYMYWVKQRPGQ GLEWIGEINPSHGGTNFNEKFKNKATLTVDKSSSTVYMQLSSLTSE DSAVYYCTRGGNYPYFAMDYWGQGTSVTVSSASTKGPSVFPLAP SSKSTSGGTAALGCLVKDYFPEPVTVSWNSGALTSGVHTFPAVLQ SSGLYSLSSVVTVPSSSLGTQTYICNVNHKPSNTKVDKRVEPKSCD KTHTCPPCPAPEAAGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSREE MTKNQVSLTCLVKGFYPSDIAVEWESNGQPENNYKTTPPVLDSDG SFFLYSKLTVDKSRWQQGNVFSCSVMHEALHNHYTQKSLSLSPGK |
| 49 | 25A11(MHAB10-PC1)LC | DIQMTQTTSSLSASLGDRVTISCRASQDISNYLNWYQQKPDGTVKL LIYYTSRLHSGVPSRFSGSGSGTDYSLTISNLEQEDIATYFCQQGNT LPWTFGGGTKLEIKRTVAAPSVFIFPPSDEQLKSGTASVVCLLNNF YPREAKVQWKVDNALQSGNSQESVTEQDSKDSTYSLSSTLTLSKA DYEKHKVYACEVTHQGLSSPVTKSFNRGEC |
| 50 | h14A043 (MHAB 10-PC2)HC | QVQLVQSGAEVKKPGSSVKVSCKASGFNIKDYYMNWVRQAPGQ GLEWIGWIDPENANTIYDPKFQGRVTITADESTSTAYMELSSLRSE DTAVYYCYGSSYRFTYWGQGTLVTVSSASTKGPSVFPLAPSSKSTS GGTAALGCLVKDYFPEPVTVSWNSGALTSGVHTFPAVLQSSGLYS LSSVVTVPSSSLGTQTYICNVNHKPSNTKVDKRVEPKSCDKTHTCP PCPAPEAAGGPSVFLFPPKPKDTLMISRTPEVTCVVVDVSHEDPEV KFNWYVDGVEVHNAKTKPREEQYNSTYRVVSVLTVLHQDWLNG KEYKCKVSNKALPAPIEKTISKAKGQPREPQVYTLPPSREEMTKNQ VSLTCLVKGFYPSDIAVEWESNGQPENNYKTTPPVLDSDGSFFLYS KLTVDKSRWQQGNVFSCSVMHEALHNHYTQKSLSLSPGK |
| 51 | h14A043 (MHAB 1 0-PC2)LC | EIVLTQSPATLSLSPGERATLSCSASSSVSYLYWYQQKPGQAPRPWI YLTSNLASGVPARFSGSGSGTDFTLTISSLEPEDFAVYYCQQWSSN PFTFGGGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC |

**Specific Models for Carrying Out the present invention**

The present invention is described by reference to the following examples which are intended to illustrate the present invention by example, rather than to limit the present invention.

Unless otherwise specified, the molecular biological experimental methods and immunoassay methods used in the present invention were carried out essentially according to the methods of J. Sambrook et al., Molecular cloning: Laboratory Manual, 2nd Ed., Cold Spring Harbor Laboratory Press, 1989, and F. M. Ausubel et al., Compiling Guidelines for Molecular Biology Experiments, 3rd Ed., John Wiley & Sons, Inc., 1995. Restriction enzymes were used according to the conditions recommended by the manufacturers of the products. Those skilled in the art know that the examples

describe the present invention by example, and are not intended to limit the scope of sought to be protected by the present invention.

Example 1. Production of CDCP1-specific antibody

1.1. Production of CDCP1-specific antibody 25A11 (MHAB10-PC1)

The variable region of 25A11 (MHAB10-PC1) antibody was derived from patent US20080008719A1. The VH and VL sequences were respectively integrated into the CH and CL sequences of Human IgG1-Kappa. The sequence of the heavy chain of the integrated antibody was set forth in SEQ ID NO: 48, and the sequence of the light chain of the antibody was set forth in SEQ ID NO: 49. After transient expression in CHO, the antibody was purified by Protein A and dissolved in PBS. The purity of the antibody solution (SEC, 280nm) was 98.887%, and the endotoxin (EU/mg) was <1. After aliquoting, the antibody was cryopreserved at -80°C. This antibody was used as a positive control antibody for subsequent screening experiments.

1.2. Production of CDCP1-specific antibody h14A043 (MHAB10-PC2)

The variable region of h14A043 (MHAB10-PC2) antibody was derived from patent US20230050380A1. The VH and VL sequences were respectively integrated into the CH and CL sequences of Human IgG1-Kappa. The sequence of the heavy chain of the integrated antibody was set forth in SEQ ID NO: 50, and the sequence of the light chain of the integrated antibody was set forth in SEQ ID NO: 51. After transient expression in CHO, the antibody was purified by Protein A and dissolved in PBS. The purity of the antibody solution (SEC, 280nm) was 79.69%. This antibody was used as the positive control antibody for the subsequent anti-human CDCP1 humanized nanobody MHAB10-16-14 activity detection experiment.

Example 2. Acquisition of anti-human CDCP1 nanobody sequence

2.1. Alpaca immunization and serum titer detection

A blank alpaca was immunized with CDCP1 antigen (Genebank ID: Q9H5V8-1). 10 mL of blood was collected before the first immunization and used as a negative serum; the first immunization was performed by subcutaneous injection at multiple points using a mixture of complete Freund's adjuvant and 0.5 mg of CDCP1 antigen in a ratio of 1:1. After that, incomplete Freund's adjuvant was mixed with 0.25 mg of CDCP1 antigen in a ratio of 1:1 and injected subcutaneously at multiple points for secondary, tertiary and fourth immunizations in the third, fifth and seventh weeks, respectively. 10 mL of peripheral blood was collected before the third and fourth immunizations, serum was separated, and immune response was monitored by ELISA. Five days after the fourth immunization, 50 mL of peripheral blood was collected for subsequent nanobody library construction (whether blood collection was performed this time was determined based on the serum immune response).

Table 2. Immunization information

| Immunization time | Operation | Adjuvant | Immunization dose |
|---|---|---|---|
| Primary immunization | Collecting 10mL of blood, primary immunization | Complete adjuvant | CDCP1 antigen, 0.5 mg |
| 13 Days after primary immunization | Secondary immunization | Incomplete adjuvant | CDCP1 antigen, 0.25 mg |
| 14 Days after second immunization | Collecting 10mL of blood, third immunization | Incomplete adjuvant | CDCP1 antigen, 0.25 mg |
| 13 Days after third immunization | Collecting 10mL of blood, fourth immunization | Incomplete adjuvant | CDCP1 antigen, 0.25 mg |
| 5 Days after fourth immunization | Collecting 10mL of blood | / | / |

2.2. Screening and identification of specific nanobodies

Peripheral blood of immunized alpacas was collected, RNA was extracted, and cDNA samples were prepared.

VHH antibody encoding genes were cloned by PCR to construct a single-domain antibody phage display library. Clones were randomly selected to identify the library capacity and diversity.

After screening and enrichment experiments were performed on the single-domain antibody phage display library using CDCP1 antigen (KACTUS, Cat.No. CDC-HM101), single clones were selected for Phage-ELISA detection after screening, and candidate clones with different amino acid sequences in CDR region were selected. Finally, 37 unique sequences were selected, and their specific information was shown in Table 3 below.

2.3. Solubility verification and antibody expression of unique sequences

The above 37 positive clone plasmids were extracted and transferred into *E. coli* Rosetta respectively, and induction expression was performed under 0.4mM IPTG at 20°C and 200rpm overnight. On the next day, centrifugation was carried out, the precipitate was resuspended in PBS, and ultrasonically broken, and centrifugation was carried out to obtain the supernatants of the 37 clones. ELISA was used to detect the binding activities of the supernatants of the 37 clones to human CDCP1 antigen ($OD_{450}$ values were shown in Table 3 below).

Table 3. $OD_{450}$ results of 37 clones

| Unique sequence | $OD_{450}$ | Unique sequence | $OD_{450}$ |
|---|---|---|---|
| MH1-6-C-1-1 | 0.086 | MH1-6-C-2-28 | 3.3 |
| MH1-6-C-1-2 | 0.072 | MH1-6-C-2-29 | 1.594 |
| MH1-6-C-1-7 | 0.093 | MH1-6-C-2-31 | 0.139 |
| MH1-6-C-1-11 | 1.36 | MH1-6-C-2-33 | 1.743 |
| MH1-6-C-1-16 | 0.148 | MH1-6-C-2-35 | 3.324 |
| MH1-6-C-1-21 | 2.372 | MH1-6-C-2-36 | 1.156 |
| MH1-6-C-1-30 | 0.52 | MH1-6-C-2-38 | 2.962 |
| MH1-6-C-1-43 | 0.29 | MH1-6-C-2-43 | 0.33 |
| MH1-6-C-1-44 | 1.632 | MH1-6-C-2-47 | 3.272 |
| MH1-6-C-1-52 | 0.065 | MH1-6-C-2-50 | 0.304 |
| MH1-6-C-1-53 | 0.32 | MH1-6-C-2-61 | 0.158 |
| MH1-6-C-1-55 | 3.233 | MH1-6-C-2-62 | 3.081 |
| MH1-6-C-1-59 | 3.272 | MH1-6-C-2-68 | 0.327 |
| MH1-6-C-1-62 | 3.243 | MH1-6-C-3-7 | 3.105 |
| MH1-6-C-1-72 | 0.414 | MH1-6-C-3-8 | 2.882 |
| MH1-6-C-1-88 | 1.926 | MH1-6-C-3-11 | 1.683 |
| MH1-6-C-2-7 | 0.154 | MH1-6-C-3-52 | 0.16 |
| MH1-6-C-2-10 | 0.252 | MH1-6-C-3-87 | 0.141 |
| MH1-6-C-2-20 | 2.266 | Positive (2PDL) | 0.64 |
| Not relevant | 0.124 | 1%M-PBS | 0.093 |

The sequences with strong binding activity to human CDCP1 were selected, the VHH sequence was integrated into the Fc (LALA) sequence (SEQ ID NO: 29) of Human IgG1, and Shanghai Baiying Biotechnology Co., Ltd. Was entrusted to perform CHO transient expression services and produce MHAB10 candidate antibodies (the correspondence between the expression numbers and the unique sequences of Nanchang Dajia Technology Co., Ltd. Were shown in the following Table 4, and the subsequent MHAB10 candidate antibodies would be described with the expression numbers, i.e., MHAB10-1~26). After expression, the antibody was purified by Protein A and dissolved in PBS. The purity of antibody solution (SEC, 280nm) was >95%, and the endotoxin (EU/mg) was <1. After aliquoting, the antibodies were stored at -80°C.

Table 4. Expression numbers corresponding to unique sequences

| Expression number | Unique sequence |
|---|---|
| MHAB10-1 | MH1-6-C-1-11 |
| MHAB10-2 | MH1-6-C-1-30 |
| MHAB10-3 | MH1-6-C-1-52 |
| MHAB10-4 | MH1-6-C-1-72 |
| MHAB10-5 | MH1-6-C-1-88 |
| MHAB10-6 | MH1-6-C-2-7 |
| MHAB10-7 | MH1-6-C-2-10 |
| MHAB10-8 | MH1-6-C-2-20 |
| MHAB10-9 | MH1-6-C-2-28 |
| MHAB10-10 | MH1-6-C-2-29 |
| MHAB10-11 | MH1-6-C-2-35 |
| MHAB10-12 | MH1-6-C-2-38 |
| MHAB10-13 | MH1-6-C-2-40 |
| MHAB10-14 | MH1-6-C-2-43 |
| MHAB10-15 | MH1-6-C-2-47 |
| MHAB10-16 | MH1-6-C-2-50 |
| MHAB10-17 | MH1-6-C-2-68 |
| MHAB10-18 | MH1-6-C-3-7 |
| MHAB10-19 | MH1-6-C-2-188 |
| MHAB10-20 | MH1-6-C-2-201 |
| MHAB 10-21 | MH1-6-C-2-216 |
| MHAB10-22 | MH1-6-C-2-226 |
| MHAB10-23 | MH1-6-C-2-245 |
| MHAB10-24 | MH1-6-C-2-254 |
| MHAB10-25 | MH1-6-C-2-263 |
| MHAB10-26 | MH1-6-C-2-276 |

Example 3. Detection of activity of anti-human CDCP1 nanobodies

3.1. ELISA binding experiment

The ELISA method was used to detect the binding activities of the MHAB10 candidate antibodies (anti-human CDCP1 nanobody Fc fusion proteins) to the human CDCP1 antigen. CDCP1, His Tag (KACTUS, Cat.No. CDC-HM101) were diluted to 0.5μg/mL using PBS, and added at 100μL/well to ELISA plate (Corning, Cat.No. 9018), and coating was carried out overnight at 2°C to 8°C. Washing was performed 3 times with PBST, 1% BSA/PBST was added to perform blocking at room temperature for 1 hour, then washing was performed 5 times with PBST, the antibody to be tested (the antibody to be tested was MHAB10, the positive control antibody was 25A11, i.e., MHAB10-PC1, the initial concentration was 10μg/mL, and subjected to 10-fold dilution) was added, incubated for 1 hour, washing was performed 7 times with PBST to wash away the unbound antibody, and Goat Anti-Human IgG Fc(HRP) (Abcam, Cat.No. ab97225) diluted at 1:50,000 was added, and incubated at room temperature for 30 minutes. After washing away the excess secondary antibody, 1-Step™ Ultra TMB-ELISA Substrate Solution (Absin, Cat.No. 9178) was added, 100μL/well, color development was performed at room temperature in the dark for 10 minutes, and then 100μL of TMB stop solution (Absin, Cat.No. abs9472) was added to stop the reaction. The absorbance values at wavelength of 450nm were read using an ELISA reader, and four-parameter curves were drawn using Graphpad. All experimental results were expressed as mean ± SEM

(standard error of mean). Prism 6 (GraphPad) software was used to plot and analyze the data.

The ELISA binding results (Fig. 1 and Fig. 2) show that the top 10 MHAB10 candidate antibodies with the strongest binding activity to human CDCP1 antigen were MHAB10-2, 3, 8, 9, 10, 11, 13, 16, 17, and 26, and the binding activities of these 10 antibodies to human CDCP1 antigen were stronger than that of the positive control antibody 25A11, i.e., MHAB10-PC1. These 10 antibodies would be tested for subsequent FACS binding activity.

3.2. FACS binding experiment

In the FACS experiment, human colorectal cancer HCT116 cells and SW480 cells were cultured in complete medium for HCT116 cells (ATCC-formulated McCoy's 5a Medium Modified, Catalog No. 30-2007+10%FBS, Sigma-Aldrich, Cat.no. F8687+1% P/S, Gibco, Cat.no. 15140-122) and complete medium for SW480 cells (ATCC-formulated Leibovitz's L-15 Medium, Catalog No. 30-2008+10%FBS, Sigma-Aldrich, Cat.no. F8687+1% P/S, Gibco, Cat.no. 15140-122), respectively. Furthermore, to prevent trypsin from cleaving fl-CDCP1, the HCT116 cells and SW480 cells were digested with Versene (Gibco, Cat.no.15040-066). Then, the human colorectal cancer HCT116 cells and SW480 cells capable of endogenously expressing human CDCP1 were used to detect the binding activities of the candidate antibodies. The candidate antibodies were serially diluted (30000ng/mL, 7500ng/mL, 1875ng/mL, 468.8ng/mL, 117.2ng/mL, 29.3ng/mL, 7.3ng/mL and 1.8ng/mL), and 100μL of secondary antibody, FITC anti-human IgG Fc Antibody (Biolegend, Cat.no. 410720), was used to detect the binding activities of the candidate antibodies. The FITC mean fluorescence intensity (MFI) was detected by flow cytometry. All experimental results were expressed as mean $\pm$ SEM (standard error of the mean). Prism 6 (GraphPad) software was used for plotting and data analysis.

The binding curves were shown in Fig. 3, in which Fig. 3A showed the results of HCT116 cells, and Fig. 3B showed the results of SW480 cells. From the FACS binding results, it could be seen that the binding activities of the MHAB10 candidate antibodies with HCT116 and SW480 cells were similar, among which MHAB10-11 and MHAB10-16 had the strongest binding activity. These two antibodies would be tested for subsequent endocytosis and endocytosis-mediated cell killing activity.

3.3. Detection of binding activities of candidate antibodies to human cleaved CDCP1 by FACS binding experiment

In the FACS experiment, PL45 cell complete culture medium (ATCC-formulated Dulbecco's Modified Eagle's Medium, Catalog No. 30-2002+10%FBS, Sigma-Aldrich, Cat.no. F8687+1% P/S, Gibco, Cat.no. 15140-122) was used to culture human pancreatic ductal adenocarcinoma PL45 cells, and then the PL45 cells endogenously expressing human Cleaved CDCP1 were used to detect the binding activities of the candidate antibodies. The antibodies to be tested were serially diluted (30000ng/mL, 7500ng/mL, 1875ng/mL, 468.8ng/mL, 117.2ng/mL, 29.3ng/mL, 7.3ng/mL and 1.8ng/mL). 100μL of secondary antibody, FITC anti-human IgG Fc Antibody (Biolegend, Cat.no. 410720), was used to detect the binding activities of the antibodies to be tested. The FITC mean fluorescence intensity (MFI) was detected by flow cytometry. All experimental results were expressed as mean $\pm$ SEM (standard error of the mean). Prism 6 (GraphPad) software was used to plot and analyze the data.

The binding curves were shown in Fig. 4. From the FACS binding results, it could be seen that the binding activities of the MHAB10 candidate antibodies to PL45 cells were consistent with the binding activity trend to human colorectal cancer HCT116 cells and SW480 cells capable of endogenously expressing human CDCP1. Among them, MHAB10-11 and MHAB10-16 had the strongest binding activity.

3.4. Endocytosis experiment

Endocytosis activity was detected using human colorectal cancer HCT116 cells and SW480 cells that could endogenously express human CDCP1. FACS buffer (PBS containing 2% FBS) was used to prepare $1\times10^6$/mL cell suspension and 10μg/mL anti-CDCP1 test antibody solutions, and the cell suspension was added to a 1.5mL EP tube, centrifuged at 1200rpm and 4°C for 3min, and the supernatant was discarded. 1.5 mL of the antibody solutions to be tested were pipetted and added to the cells, respectively, incubated on ice for 30 min, and washed four times with 200 μL/well of pre-cooled FACS buffer. The cells were resuspended with 1.5 mL of complete medium, mixed and added to 96-well plates at 100 μL/well, and incubated at 4°C and 37°C for 0 h, 1 h, 2 h, and 4 h (duplicate wells), respectively. After incubation, centrifugation was carried out at 1200 rpm and 4°C for 3 min, 100 μL of FITC anti-human IgG Fc Antibody diluent (Biolegend, Cat.no. 410720) was added, and incubated at 4°C for 30 min, the unbound secondary antibody was washed off, and a flow cytometry was used to detect FITC fluorescence intensity (Mean Fluorescence Intensity, MFI) to calculate the internalization ratio. The calculation formula was as follows:

$$\text{Internalization (\%)} = 100\% - MFI_T / MFI_{T0} * 100\%$$

All experimental results were expressed as mean ± SEM (standard error of the mean). Prism 6 (GraphPad) software was used for plotting and data analysis.

The internalization percentage curves were shown in Fig. 5, in which Fig. 5A showed the results of HCT116 cells, and Fig. 5B showed the results of SW480 cells. The endocytosis results showed that at 37°C, the MHAB10 candidate antibodies had good endocytosis activity in HCT116 and SW480 cells, and the trend was consistent, that was, the endocytosis activity of MHAB10-16 was significantly stronger than that of MHAB10-11 and that of the positive control antibody (25A11) (MHAB10-PC1).

3.5. Experiment of detecting antibody endocytosis-mediated cell killing by Fab-ZAP method

Human colorectal cancer HCT116 cells were digested and resuspended in complete culture medium (ATCC-formulated McCoy's 5a Medium Modified, Catalog No. 30-2007+10% FBS, Sigma-Aldrich, Cat.no. F8687+1% P/S, Gibco, Cat.no. 15140-122), and the cell density was adjusted to 1E4 cells/mL. 50μL was added to each well of a 96-well cell culture plate, and the cell culture plate was placed in a 37°C cell culture incubator for 16 hours. On the second day, ZAP diluent containing 9nM ZAP-Fab was prepared with culture medium, and then the antibody was gradiently diluted with ZAP diluent to obtain 1.6nM to 0.5pM (6 concentration gradients, 1:5 dilution) working solutions. After incubation at 37°C for 15min, the working solution was added to the cell culture plate, 50μL per well, and mixed, then the cell culture plate was placed in a 37°C cell culture incubator for further incubation for 120h. 7.5μL of Triton-X 100 was added to the cell wells not treated with antibody-Fab-ZAP in advance, and incubated for 30min, and this well was used as a positive control. The cell wells (NT) not treated with antibody-Fab-ZAP or Triton-X100 were used as negative controls. Then 20μL of MTS (Promega, Cat: G3598B) was added to each well of the cell culture plate, incubated in a 37°C cell culture incubator for 2h, and the data was read after shaking for 10s in an ELISA reader, and the detection wavelength was A490. The cell killing efficiency was calculated using the following formula: killing rate % = 100% - $(OD_{sample} - OD_{Triton\text{-}X100})/(OD_{NT} - OD_{Triton\text{-}X100})$*100%.

All experimental results were expressed as mean ± SEM (standard error of the mean). Prism 6 (GraphPad) software was used for plotting and data analysis.

The results of endocytosis-mediated cell killing were shown in Table 5 and Fig. 6. MHAB10-16-Fab-ZAP had good killing activity against HCT116 cells. Based on the FACS binding and endocytosis results, MHAB10-16 would be humanized and subsequently screened.

Table 5. Endocytosis-mediated cell killing results

| | MHAB10-16 | Isotype |
|---|---|---|
| Bottom | 4.235 | N/A |
| Top | 61.50 | N/A |
| $EC_{50}$ (pM) | 13.83 | N/A |

<u>Example 4. Humanization and activity detection of anti-human CDCP1 nanobody</u>

4.1. Preparation of MHAB10-16 nanobody and humanized nanobody

After sequencing and analysis, the CDR sequence (defined according to the IMGT numbering system (Lefranc et al., Dev. Comparat. Immunol. 27:55-77, 2003)), VHH sequence and complete sequence of MHAB10-16 nanobody were obtained. The specific sequence information was shown in Table 1 and Table 6 below.

Table 6. MHAB10-16 nanobody sequence information

| Numbering system | CDR1 | CDR2 | CDR3 | VHH |
|---|---|---|---|---|
| Kabat | SEQ ID NO: 1 | SEQ ID NO: 2 | SEQ ID NO: 3 | SEQ ID NO: 10 |
| Chothia | SEQ ID NO: 4 | SEQ ID NO: 5 | SEQ ID NO: 6 | SEQ ID NO: 10 |
| IMGT | SEQ ID NO: 7 | SEQ ID NO: 8 | SEQ ID NO: 9 | SEQ ID NO: 10 |

Furthermore, by analyzing the sequence of the original camel-derived antibody MHAB10-16 in the database, the humanized sequence Germeline to be referred was to determined, and humanized sequences were designed. After the first round of humanization, their degree of humanization was at least 82%, and they were constructed into nanobody-Fc fusion proteins (a total of 10 molecules from MHAB10-16-1 to MHAB10-16-10 were constructed), and transient expression in CHO cells was performed. The second round of humanization was carried out according to the degree of humanization, affinity and expression amount. After the second round of humanization, they were constructed into nanobody-Fc fusion proteins (a total of 7 molecules from MHAB10-16-11 to MHAB10-16-17 were constructed), and transient expression in CHO cells was performed and their binding activity was verified.

A total of 17 humanized nanobodies were prepared, named MHAB10-16-1 to MHAB10-16-17, and their specific sequence information was shown in Table 1.

4.2. ELISA binding experiment

The binding activities of MHAB10-16 humanized sequences to human CDCP1antigen were detected by ELISA. CDCP1, His Tag (KACTUS, Cat.No. CDC-HM101) were diluted to 0.5μg/mL using PBS, and added at 100μL/well to ELISA plate (Corning, Cat.No. 9018), and coating was performed overnight at 2°C to 8°C. Washing was performed 3 times with PBST, blocking was performed with 1% BSA/PBST at room temperature for 1 hour, then washing was performed 5 times with PBST, MHAB10-16 humanized antibodies (antibody numbers were MHAB10-16-1~8, positive control antibody was 25A11, i.e., MHAB10-PC1, they had an initial concentration of 10μg/mL, and were subjected to 10-fold dilution) were added, incubated for 1 hour, and then washing was performed 7 times with PBST to remove unbound antibodies, and Goat Anti-Human IgG Fc(HRP) (Abcam, Cat.No. ab97225) diluted at 1:50,000 was added, and incubated at room temperature for 30 minutes. After washing away the excess secondary antibody, 1-Step™ Ultra TMB-ELISA Substrate Solution (Absin, Cat.No. 9178) was added, 100μL/well, and color development was carried out at room temperature in the dark for 15min, and then 100μL of TMB stop solution (Absin, Cat.No. abs9472) was added to stop the reaction. The absorbance values at wavelength of 450nm were read using an ELISA reader, and four-parameter curves were drawn using Graphpad.

All experimental results were expressed as mean ± SEM (standard error of the mean). Prism 6 (GraphPad) software was used to plot and analyze the data.

From the ELISA binding results (Fig. 7A, Fig. 7B and Table 7), the binding activities of MHAB10-16 humanized antibodies to human CDCP1 antigen were basically equivalent to that of the parental MHAB10-16, and all of them were higher than that of the positive control antibody 25A11, i.e., MHAB10-PC1.

The ELISA binding $EC_{50}$ results of MHAB10-16 and MHAB10-16-1 to MHAB10-16-8 were shown in Table 7 below.

Table 7. ELISA binding $EC_{50}$ results

| Ab | MHAB10-1 6 | MHAB10-1 6-1 | MHAB10-1 6-2 | MHAB10-1 6-3 | MHAB10-1 6-4 | MHAB10-1 6-5 |
|---|---|---|---|---|---|---|
| bottom | 0.04478 | 0.04434 | 0.04546 | 0.0343 | 0.04623 | 0.03866 |
| Top | 1.791 | 2.177 | 2.16 | 2.153 | 2.178 | 2.169 |
| $EC_{50}$ (ng/mL) | 5.446 | 4.112 | 3.625 | 2.981 | 3.823 | 3.601 |
| MW (kDa) | 76kDa | 76kDa | 76kDa | 76kDa | 76kDa | 76kDa |
| Isoelectric point | 7.68 | 7.27 | 7.27 | 7.75 | 7.75 | 7.27 |
| Expression (mg/L) | 366.5 | 88.0 | 102.0 | 112.0 | 108.0 | 97.0 |
| Purity (SEC, 280nm) | 98.663% | 99.236% | 99.307% | 99.306% | 99.287% | 99.324% |

Continued Table 7. ELISA binding $EC_{50}$ results

| Ab | MHAB10-1 6-6 | MHAB10-1 6-7 | MHAB10-1 6-8 | 25A11 | MHAB10-1 6-9* | MHAB10-1 6-10* |
|---|---|---|---|---|---|---|
| bottom | 0.05571 | 0.04918 | 0.04826 | 0.03954 | | |
| Top | 2.06 | 2.065 | 2.061 | 1.901 | | |
| $EC_{50}$ (ng/mL) | 4.558 | 4.85 | 5.264 | 11.47 | | |
| MW (kDa) | 76kDa | 76kDa | 76kDa | 146kDa | 76kDa | 76kDa |

(continued)

| Ab | MHAB10-1 6-6 | MHAB10-1 6-7 | MHAB10-1 6-8 | 25A11 | MHAB10-1 6-9* | MHAB10-1 6-10* |
|---|---|---|---|---|---|---|
| Isoelectric point | 7.75 | 7.75 | 7.27 | 7.85 | 7.7 | 7.27 |
| Expression (mg/L) | 98.0 | 121.0 | 171.0 | 461.1 | 261.0 | 91.0 |
| Purity (SEC, 280nm) | 99.385% | 99.324% | 99.686% | 98.89% | 99.386% | 99.662% |
| Note: MHAB10-16-9* and MHAB10-16-10* were intermediate sequences between the first round of humanization and the second round of humanization to explore the change in expression (the sequences were two variants of MHAB10-16-1 after mutation, respectively, and the sequence information was shown in Table 1). ELISA binding activity was not detected. | | | | | | |

4.3. FACS binding experiment

In the FACS experiment, human colorectal cancer HCT116 cells endogenously expressing human CDCP1 were used to detect the binding activities of MHAB10-16 humanized antibodies. The antibodies to be tested were serially diluted (7500ng/mL, 1875ng/mL, 375ng/mL, 75ng/mL, 15ng/mL, 3ng/mL and 0.6ng/mL). 100μL of Alexa Fluor ® 488 AffiniPure Goat Anti-Human IgG(H+L) secondary antibody (YEASEN, Cat.no. 33126ES60) was used to detect the binding activities of the antibodies to be tested. The mean fluorescence intensity (MFI) of binding secondary antibody was detected by flow cytometry.

All experimental results were expressed as mean ± SEM (standard error of the mean). Prism 6 (GraphPad) software was used for plotting and data analysis.

The binding curves were shown in Fig. 8, and the $EC_{50}$ results were shown in Table 8. The FACS binding results showed that the binding activities of MHAB10-16 humanized antibodies to HCT116 cells were comparable to that of the parental MHAB10-16. Based on the $EC_{50}$ and humanization level, the three antibodies with the best development potential (MHAB10-16-3, 4, and 7) were selected for subsequent endocytosis and endocytosis-mediated cell killing activity testing.

The ELISA binding $EC_{50}$ results of MHAB10-16 and MHAB10-16-1 to MHAB10-16-8 were shown in Table 8 below.

Table 8. FACS binding $EC_{50}$ results

| Ab | MHAB10-16 | MHAB10-16-1 | MHAB10-16-2 | MHAB10-16-3 | MHAB10-16-4 |
|---|---|---|---|---|---|
| bottom | 857.2 | 1131 | 1183 | 1098 | 699.7 |
| Top | 61580 | 62167 | 63376 | 64039 | 64729 |
| $EC_{50}$ (ng/mL) | 64.16 | 78.41 | 80.03 | 73.95 | 67.49 |

Continued Table 8. FACS binding $EC_{50}$ results

| Ab | MHAB10-16-5 | MHAB10-16-6 | MHAB10-16-7 | MHAB10-16-8 | Isotype |
|---|---|---|---|---|---|
| bottom | 984.4 | 1087 | 1208 | 1012 | N/A |
| Top | 63037 | 63158 | 62316 | 62207 | N/A |
| $EC_{50}$ (ng/mL) | 71.96 | 66.12 | 82.93 | 78.15 | N/A |

4.4. Endocytosis experiment

Endocytosis activity was detected using human colorectal cancer HCT116 cells capable of endogenously expressing human CDCP1. $1\times10^6$/mL cell suspension and 10μg/mL solutions of anti-CDCP1 antibodies to be tested were prepared using FACS buffer (PBS containing 2% FBS), and the cell suspension was added to 1.5mL EP tubes, centrifuged at 1200rpm and 4°C for 3min, and the supernatant was discarded. 1.5mL of the solutions of antibodies to be tested were added to the cells, respectively, incubated on ice for 30min, and washed four times with pre-cooled 200μL/well FACS buffer. The cells were resuspended with 1.5mL of complete medium, mixed well and added to 96-well plates, 100μL/well, and incubated at 4°C and 37°C for 0h, 1h, 2h and 4h (double replicate wells), respectively. After incubation,

centrifugation was performed at 1200rpm, 4°C for 3min, then 100μL of Alexa Fluor ® 488 AffiniPure Goat Anti-Human IgG(H+L) secondary antibody (YEASEN, Cat.no. 33126ES60) diluent was added, incubated at 4°C for 30min. After the unbound secondary antibody was washed off, a flow cytometry was used to detect FITC fluorescence intensity (Mean Fluorescence Intensity, MFI), and the internalization ratio was calculated. The calculation formula was as follows:

$$\text{Internalization (\%)} = 100\% - MFI_T / MFI_{T0} * 100\%$$

All experimental results were expressed as mean ± SEM (standard error of the mean). Prism 6 (GraphPad) software was used to plot and analyze the data.

The endocytosis percentage curves were shown in Fig. 9. From the endocytosis results, it could be seen that at 37°C, the MHAB10-16 humanized antibody had good endocytosis activity in HCT116 cells, and the endocytosis activities of MHAB10-16-3, 4, and 7 were basically equivalent to that of parental MHAB10-16, and all were higher than that of the positive control antibody 25A11, i.e., MHAB10-PC1.

4.5. Experiment of detecting antibody endocytosis-mediated cell killing by Fab-ZAP method

Human colorectal cancer HCT116 cells were digested and resuspended in complete culture medium (ATCC-formulated McCoy's 5a Medium Modified, Catalog No. 30-2007+10% FBS, Sigma-Aldrich, Cat.no. F8687+1% P/S, Gibco, Cat.no. 15140-122), and the cell density was adjusted to 1E4 cells/mL. 50μL of the cells was added to each well of a 96-well cell culture plate, and the cell culture plate was placed in a 37°C cell culture incubator for 16 hours. On the second day, ZAP diluent containing 9nM ZAP-Fab was prepared with culture medium, and then the antibodies were gradiently diluted with ZAP diluent to obtain 1.6nM to 0.5pM (6 concentration gradients, 1:5 dilution) working solutions. After incubation at 37°C for 15min, the working solutions were added to the cell culture plate, 50μL per well; and after mixing, the cell culture plate was placed in a 37°C cell culture incubator for further incubation for 120h. 7.5μL of Triton-X 100 was added to the cell wells not treated with antibody-Fab-ZAP in advance, and incubated for 30min, and this well was used as a positive control. The cell wells (NT) not treated with antibody-Fab-ZAP or Triton-X100 were used as negative controls. Then 20μL of MTS (Promega, Cat: G3598B) was added to each well of the cell culture plate, incubated in a 37°C cell culture incubator for 2h, and the data was read after shaking for 10s in an ELISA reader, and the detection wavelength was A490. The cell killing efficiency was calculated using the following formula:

$$\text{killing rate \%} = 100\% - (OD_{sample} - OD_{Triton\text{-}X100})/(OD_{NT} - OD_{Triton\text{-}X100}) * 100\%.$$

All experimental results were expressed as mean ± SEM (standard error of the mean). Prism 6 (GraphPad) software was used for plotting and data analysis.

The results of endocytosis-mediated cell killing were shown in Table 9 and Fig. 10. Compared with parental MHAB10-16, the MHAB10-16 humanized antibodies exhibited similar killing activity against HCT116 cells.

Table 9. Endocytosis-mediated cell killing results

| | MHAB10-16 | MHAB10-16-3 | MHAB 10-16-4 | MHAB10-16-7 | Isotype |
|---|---|---|---|---|---|
| Bottom | 9.830 | 13.08 | 12.95 | 14.15 | N/A |
| Top | 67.60 | 75.57 | 73.16 | 77.55 | N/A |
| $EC_{50}$ (pM) | 16.82 | ~ 31.79 | 21.22 | 24.27 | N/A |

Example 5. Second round humanization and activity detection of anti-human CDCP1 nanobodies

5.1. ELISA binding experiment

The binding activities of MHAB10-16 humanized sequences to human CDCP1 antigen were detected by ELISA. CDCP1, His Tag (KACTUS, Cat.No. CDC-HM101) were diluted to 0.5μg/mL with PBS, and added at 100μL/well to ELISA plates (Corning, Cat.No. 9018), and coating was performed overnight at 2°C to 8°C. Washing was performed 3 times with PBST, 1% BSA/PBST was added to perform blocking at room temperature for 1h, then washing was performed 5 times with PBST, the MHAB10-16 humanized antibodies (antibody numbers were MHAB10-16-11~17, positive control antibody was 25A11, i.e., MHAB10-PC1, they had an initial concentration of 10μg/mL, and subjected to 10-fold gradient dilution) were added, incubated for 1h, then washing was performed 7 times with PBST to wash away the unbound antibodies, and Goat Anti-Human IgG Fc(HRP) (Abcam, Cat.No. ab97225) diluted at 1:50,000 was added, and incubated at room

temperature for 30min. After the excess secondary antibody was washed away, 1-Step™ Ultra TMB-ELISA Substrate Solution (Absin, Cat.No. 9178) was added, 100μL/well, and color development was performed at room temperature in the dark for 15min, and then 100μL of TMB stop solution (Absin, Cat.No. abs9472) was added to stop the reaction. The absorbance values at wavelength of 450nm were read using an ELISA reader, and four-parameter curves were drawn using Graphpad.

All experimental results were expressed as mean ± SEM (standard error of the mean). Prism 6 (GraphPad) software was used to plot and analyze the data.

From the ELISA binding results (Fig. 11 and Table 10), it could be seen that the binding activities of MHAB10-16 humanized antibodies to human CDCP1 antigen were basically equivalent to that of parental MHAB10-16, and all of them were higher than that of the positive control antibody 25A11, i.e., MHAB10-PC1.

The ELISA binding $EC_{50}$ results of MHAB10-16 and MHAB10-16-11 to MHAB10-16-17 were shown in Table 10 below.

Table 10. ELISA binding $EC_{50}$ results

| Ab | MHAB10-16 | MHAB10-16-11 | MHAB 10-16-12 | MHAB 10-16-13 | MHAB 10-16-14 |
|---|---|---|---|---|---|
| bottom | 0.04939 | 0.05216 | 0.05168 | 0.05401 | 0.0514 |
| Top | 1.607 | 1.588 | 1.598 | 1.594 | 1.584 |
| $EC_{50}$ (ng/mL) | 2.969 | 4.769 | 4.169 | 4.868 | 4.283 |
| Isoelectric point | 7.68 | 7.7 | 8.07 | 8.07 | 7.7 |
| Expression (mg/L) | 366.5 | 85 | 99 | 155 | 241 |
| Purity (SEC, 280nm) | 98.66% | 99.07% | 98.91% | 99.01% | 99.28% |

Continued Table 10. ELISA binding $EC_{50}$ results

| Ab | MHAB10-16-15 | MHAB10-16-16 | MHAB10-16-17 | 25A11 | Isotype |
|---|---|---|---|---|---|
| bottom | 0.051 | 0.04907 | 0.04786 | 0.041 | N/A |
| Top | 1.576 | 1.562 | 1.563 | 1.355 | N/A |
| $EC_{50}$ (ng/mL) | 4.89 | 5.473 | 5.372 | 10.13 | N/A |
| Isoelectric point | 8.07 | 8.07 | 7.7 | 7.85 | N/A |
| Expression (mg/L) | 202 | 165 | 184 | 461.1 | N/A |
| Purity (SEC, 280nm) | 99.22% | 99.15% | 98.23% | 98.89% | N/A |

5.2. FACS binding experiment

In the FACS experiment, the binding activities of MHAB10-16 humanized antibodies were detected using human colorectal cancer HCT116 cells endogenously expressing human CDCP1. The antibodies to be tested were serially diluted (30000ng/mL, 7500ng/mL, 1875ng/mL, 375ng/mL, 75ng/mL, 15ng/mL, 3ng/mL, 0.6ng/mL, 0.12ng/mL and 0.024ng/mL), 100μL of Alexa Fluor ® 488 AffiniPure Goat Anti-Human IgG(H+L) secondary antibody (YEASEN, Cat. no. 33126ES60) was used to detect the binding activities of the antibodies to be tested, and the mean fluorescence intensity (MFI) of the bound secondary antibody was detected by flow cytometry.

All experimental results were expressed as mean ± SEM (mean standard error). Prism 6 (GraphPad) software was used to draw graphs and analyze data.

The binding curves were shown in Fig. 12, and the EC50 results were shown in Table 11. From the FACS binding results, it could be seen that the binding activities of MHAB10-16 humanized antibodies to HCT116 cells were not significantly weakened as compared to that of the parental MHAB10-16. Based on the comprehensive expression level, $EC_{50}$ and humanization level, the four antibodies with the best development potential (MHAB10-16-13, 14, 15, 16) were selected for subsequent endocytosis and endocytosis-mediated cell killing activity detection.

The FACS binding $EC_{50}$ results of MHAB10-16 and MHAB10-16-11 to MHAB10-16-17 were shown in Table 11 below.

Table 11. FACS binding $EC_{50}$ results

| Ab | MHAB10-16 | MHAB10-16-11 | MHAB10-16-12 | MHAB10-16-13 | MHAB 10-16-14 |
|---|---|---|---|---|---|
| bottom | 410.0 | 817.8 | 679.6 | 637.1 | 818.7 |
| Top | 56199 | 53442 | 53916 | 54198 | 52921 |
| $EC_{50}$ (ng/mL) | 55.34 | 69.47 | 74.15 | 72.61 | 80.91 |

Continued Table 11. FACS binding $EC_{50}$ results

| Ab | MHAB10-16-15 | MHAB10-16-16 | MHAB10-16-17 | Isotype |
|---|---|---|---|---|
| bottom | 695.1 | 863.8 | 889.2 | N/A |
| Top | 53893 | 53396 | 52859 | N/A |
| EC50(ng/mL) | 96.25 | 92.19 | 118.9 | N/A |

5.3. Endocytosis experiment

Endocytosis activity was detected using human colorectal cancer HCT116 cells capable of endogenously expressing human CDCP1. FACS buffer (PBS containing 2% FBS) was used to prepare $1\times10^6$/mL cell suspension and 10μg/mL solutions of anti-CDCP1 antibodies to be tested, the cell suspension was added to 1.5mL EP tubes, centrifuged at 1200rpm, 4°C for 3min, and the supernatant was discarded. 1.5mL of each of the solutions of antibodies to be tested was pipetted and added to the cells, incubated on ice for 30min, and washed four times with pre-cooled 200μL/well FACS buffer. The cells were resuspended with 1.5mL of complete culture medium, mixed well, and added to 96-well plates, 100μL/well, incubated at 4°C and 37°C for 0h, 1h, 2h and 4h (double replicate wells), respectively. After incubation, centrifugation was performed at 1200rpm and 4°C for 3min, 100μL of Alexa Fluor® 488 AffiniPure Goat Anti- Human IgG (H+L) secondary antibody (YEASEN, Cat.no. 33126ES60) diluent was added, and incubated at 4°C for 30 min; after the unbound secondary antibody was washed off, and a flow cytometry was used to detect FITC fluorescence intensity (Mean Fluorescence Intensity, MFI), and the internalization ratio was calculated by the calculation formula as follows:

$$\text{Internalization } (\%) = 100\% - MFI_T / MFI_{T0} * 100\%$$

All experimental results were expressed as mean ± SEM (standard error of the mean). Prism 6 (GraphPad) software was used for plotting and data analysis.

The internalization percentage curves were shown in Fig. 13. The endocytosis results showed that at 37°C, the MHAB10-16 humanized antibodies had good endocytosis activity in HCT116 cells. The endocytosis activities of MHAB10-16-13, 14, 15, and 16 were similar, and the endocytosis activities of MHAB10-16-13, 14, 15, and 16 were basically equivalent to that of the parental MHAB10-16, and all of them were significantly higher than that of the positive control antibody 25A11, i.e., MHAB10-PC1.

5.4. Fab-ZAP method to detect antibody endocytosis-mediated cell killing experiment

Human colorectal cancer HCT116 cells were digested and resuspended in complete culture medium (ATCC-formulated McCoy's 5a Medium Modified, Catalog No. 30-2007+10% FBS, Sigma-Aldrich, Cat.no. F8687+1% P/S, Gibco, Cat.no. 15140-122), and the cell density was adjusted to 1E4 cells/mL. 50μL was added to each well of a 96-well cell culture plate, and the cell culture plate was placed in a 37°C cell culture incubator for 16 hours. On the second day, ZAP diluent containing 9nM ZAP-Fab was prepared with culture medium, and then the antibodies were gradiently diluted with ZAP diluent to obtain 1.6nM to 0.5pM (6 concentration gradients, 1:5 dilution) working solutions. After incubation at 37°C for 15min, the working solutions were added to the cell culture plate, 50μL per well, and after mixing, the cell culture plate was placed in a 37°C cell culture incubator for further incubation for 120h. 7.5μL of Triton-X 100 was added to the cell wells not treated with antibody-Fab-ZAP in advance, and incubated for 30min, and this well was used as a positive control. The cell wells (NT) not treated with antibody-Fab-ZAP or Triton-X100 were used as negative controls. Then 20μL of MTS (Promega, Cat: G3598B) was added to each well of the cell culture plate, incubated in a 37°C cell culture incubator for 2h, and the data was read after shaking for 10s in an ELISA reader, and the detection wavelength was A490. The cell killing efficiency was calculated using the following formula:

$$\text{killing rate \%} = 100\% - (OD_{sample} - OD_{Triton\text{-}X100})/(OD_{NT} - OD_{Triton\text{-}X100})*100\%.$$

All experimental results were expressed as mean ± SEM (standard error of the mean). Prism 6 (GraphPad) software was used to plot and analyze the data.

The endocytosis-mediated cell killing curves were shown in Fig. 14. The endocytosis activities of MHAB10-16-13, 14, 15, and 16 were basically equivalent to that of the parental MHAB10-16.

Example 6. Activity detection of anti-human CDCP1 humanized nanobody MHAB 10-16-14

6.1. ELISA binding experiment

The binding activities of anti-human CDCP1 humanized nanobody MHAB10-16-14 and negative control Isotype to CDCP1 antigens of different species were detected by ELISA. Human CDCP1, His Tag (KACTUS, Cat.No. CDC-HM101) and rhesus monkey CDCP1, His Tag (KACTUS, Cat.No. CDC-CM101) were diluted to 0.5μg/mL with PBS, and added to ELISA plates (Corning, Cat.No. 9018) at 100μL/well, and coating was performed overnight at 2°C to 8°C. The plates were washed with PBST for 3 times, blocked with 1% BSA/PBST for 1h at room temperature, then washed with PBST for 5 times, and humanized antibody MHAB10-16-14 (negative control antibody was Isotype antibody, initial concentration was 10μg/mL, 10-fold gradient dilution was carried out) was added. After incubation for 1h, the plates were washed with PBST for 7 times to wash away unbound antibodies, and Goat Anti-Human IgG Fc(HRP) (Abcam, Cat.No. ab97225) diluted at 1:50,000 was added and incubated at room temperature for 30min. After the excess secondary antibody was washed away, 1-Step™ Ultra TMB-ELISA Substrate Solution, (Absin, Cat.No. 9178), was added, 100μL/well, and color development was carried out at room temperature in the dark for 15min, and then 100μL of TMB stop solution (Absin, Cat.No. abs9472) was added to stop the reaction. An ELISA reader was used to read the absorbance values at wavelength of 450nm, and Graphpad was used to draw four-parameter curves.

All experimental results were expressed as mean ± SEM (standard error of the mean). Prism 6 (GraphPad) software was used to draw graphs and perform data analysis.

From the ELISA binding results (Table 12 and Fig. 15), it could be seen that MHAB10-16-14 could specifically bind to human and rhesus monkey CDCP1 antigens, and the binding activities were comparable.

Table 12. ELISA binding $EC_{50}$ results

| Antibody | MHAB10-16-14 | | Isotype | |
|---|---|---|---|---|
| Antigen species | Human | Rhesus monkey | Human | Rhesus monkey |
| Bottom | 0.04535 | 0.04412 | N/A | N/A |
| Top | 1.795 | 1.768 | N/A | N/A |
| $EC_{50}$ (pM) | 3.372 | 4.052 | N/A | N/A |

6.2. FACS binding experiment

In the FACS experiment, human colorectal cancer HCT116, human colorectal cancer SW480, human pancreatic ductal adenocarcinoma PL45, human colorectal cancer HT-29 and human prostate cancer LNCaP cells that could endogenously express human CDCP1 were used to detect the binding activity of humanized antibody MHAB10-16-14 (positive control antibody was h14A043, i.e., MHAB10-PC2). The antibodies to be tested were serially diluted (30000ng/mL, 6000ng/mL, 1200ng/mL, 240ng/mL, 48ng/mL, 9.6ng/mL, 1.92ng/mL and 0.384ng/mL), 100μL of secondary antibody, FITC anti-human IgG Fc Antibody (Biolegend, Cat.no. 366916), was used to detect the binding activities of the antibodies to be tested, and the mean fluorescence intensity (MFI) of the bound secondary antibody was detected by a flow cytometry.

All experimental results were expressed as mean ± SEM (standard error of the mean). Prism 6 (GraphPad) software was used for plotting and data analysis.

The binding curves were shown in Fig. 16 to Fig. 20 and Table 13. From the FACS binding results, it could be seen that MHAB10-16-14 could specifically bind to different human tumor cells (taking human colorectal cancer HCT116, human colorectal cancer SW480, human pancreatic ductal adenocarcinoma PL45, human colorectal cancer HT-29 and human prostate cancer LNCaP cells as examples), and the all binding activities of MHAB10-16-14 were stronger than those of the positive control antibody h14A043.

Table 13. FACS combined $EC_{50}$ results

| | | MHAB10-16-14 | h14A043 | Isotype |
|---|---|---|---|---|
| $EC_{50}$ (ng/mL) | HCT116 | 139.1 | 11868 | N/A |
| | SW480 | 95.80 | 2420 | N/A |
| | PL45 | 167.0 | N/A | N/A |
| | HT-29 | 153.3 | N/A | N/A |
| | LNCaP | 206.5 | 2207 | N/A |

6.3. Experiment of detecting MHAB10-16-14 antibody endocytosis-mediated cell killing by Fab-ZAP method

Human colorectal cancer HCT116 cells were digested and resuspended with complete culture medium (ATCC-formulated McCoy's 5a Medium Modified, Catalog No. 30-2007+10%FBS, Sigma-Aldrich, Cat.no. F8687+1% P/S, Gibco, Cat.no. 15140-122), the cell density was adjusted to 1E4 cells/mL, and the cells were added to a 96-well cell culture plate, 50 μL per well, and the cell culture plate was placed in a 37°C cell culture incubator for incubation for 16 hours. On the second day, ZAP diluent containing 9nM ZAP-Fab was prepared with culture medium, and then the antibody was gradiently diluted with ZAP diluent to obtain 2nM to 0.16pM (the positive control antibody was h14A043, i.e., MHAB10-PC2; the concentration was set as follows: 8 concentration gradients, 1:5 dilution at the first three points, 1:2 dilution twice, and then 1:5 dilution) working solutions. After incubation at 37°C for 15 minutes, the working solutions were added to the cell culture plate, 50 μL per well, and mixed well, then the cell culture plate was placed in a 37°C cell culture incubator for further incubation for 120 hours. 7.5μL of Triton-X 100 was added to a cell well not treated with antibody-Fab-ZAP in advance, and incubated for 30 minutes, and this well was used as a positive control. The cell wells (NT) not treated with antibody-Fab-ZAP or Triton-X100 were used as negative controls. Then 20μL of MTS (Promega, Cat: G3598B) was added to each well of the cell culture plate, incubated in a 37°C cell culture incubator for incubation for 2 hours, the data were read after shaking for 10s in an ELISA reader, and the detection wavelength was A490. The cell killing efficiency was calculated using the following formula:

$$\text{killing rate } \% = 100\% - (OD_{sample} - OD_{Triton\text{-}X100})/(OD_{NT} - OD_{Triton\text{-}X100})*100\%.$$

All experimental results were expressed as mean ± SEM (standard error of the mean). Prism 6 (GraphPad) software was used for plotting and data analysis.

The endocytosis-mediated cell killing curves were shown in Fig. 21 and Table 14. The killing activity of MHAB10-16-14 on HCT116 was significantly stronger than that of the positive control antibody h14A043.

Table 14. Endocytosis-mediated cell killing results

| | MHAB10-16-14 | h14A043 | Isotype |
|---|---|---|---|
| Bottom | 20.75 | 22.04 | N/A |
| Top | 51.66 | 39.90 | N/A |
| $EC_{50}$ (pM) | 21.72 | 40.95 | N/A |

Although the specific models of the present invention have been described in detail, those skilled in the art will understand that various modifications and changes can be made to the details based on all the teachings that have been disclosed, and these changes are within the scope of protection of the present invention. All of the present invention is given by the appended claims and any equivalents thereof.

## Claims

**1.** A single-domain antibody or antigen-binding fragment thereof capable of specifically binding to CUB domain-containing protein 1 (CDCP1), wherein the single-domain antibody or antigen-binding fragment thereof comprises:

(a) a CDR1, which has: a sequence as set forth in any one of SEQ ID NOs: 1, 4, 7, or a sequence having a substitution, deletion or addition of one or more amino acids (e.g., a substitution, deletion or addition of 1, 2 or 3

amino acids) as compared to the sequence as set forth in any one of SEQ ID NOs: 1, 4, 7;
(b) a CDR2, which has: a sequence as set forth in any one of SEQ ID NOs: 2, 5, 8, or a sequence having a substitution, deletion or addition of one or more amino acids (e.g., a substitution, deletion or addition of 1, 2 or 3 amino acids) as compared to the sequence as set forth in any one of SEQ ID NOs: 2, 5, 8; and
(c) a CDR3, which has: a sequence as set forth in any one of SEQ ID NOs: 3, 6, 9, or a sequence having a substitution, deletion or addition of one or more amino acids (e.g., a substitution, deletion or addition of 1, 2 or 3 amino acids) as compared to the sequence as set forth in any one of SEQ ID NOs: 3, 6, 9;

preferably, the substitution is a conservative substitution;
preferably, the single-domain antibody or antigen-binding fragment thereof comprises:

(1) a CDR1 as set forth in SEQ ID NO: 1; a CDR2 as set forth in SEQ ID NO: 2; and a CDR3 as set forth in SEQ ID NO: 3;
(2) a CDR1 as set forth in SEQ ID NO: 4; a CDR2 as set forth in SEQ ID NO: 5; and a CDR3 as set forth in SEQ ID NO: 6; or,
(3) a CDR1 as set forth in SEQ ID NO: 7; a CDR2 as set forth in SEQ ID NO: 8; and a CDR3 as set forth in SEQ ID NO: 9.

**2.** A single-domain antibody or antigen-binding fragment thereof capable of specifically binding to CUB domain-containing protein 1 (CDCP1), wherein the single-domain antibody or antigen-binding fragment thereof comprises: 3 CDRs contained in a heavy chain variable region (VHH) as set forth in any one of SEQ ID NOs: 10-27; preferably, the 3 CDRs contained in the VHH are defined by the Kabat, IMGT or Chothia numbering system.

**3.** The single-domain antibody or antigen-binding fragment thereof according to claim 1 or 2, which comprises an amino acid sequence selected from the following:

(i) a sequence as set forth in any one of SEQ ID NOs: 10-27;
(ii) a sequence having a substitution, deletion or addition of one or more amino acids (e.g., a substitution, deletion or addition of 1, 2, 3, 4, 5, 6, 7 or 8 amino acids) as compared to the sequence as set forth in any one of SEQ ID NOs: 10-27; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in any one of SEQ ID NOs: 10-27;

preferably, the substitution is a conservative substitution.

**4.** A polypeptide construct specifically binding to CDCP1, comprising the single-domain antibody or antigen-binding fragment thereof according to any one of claims 1 to 3, and an immunoglobulin Fc domain;

preferably, the immunoglobulin Fc domain is connected to the N-terminal and/or C-terminal (e.g., C-terminal) of the single-domain antibody or antigen-binding fragment thereof optionally via a peptide linker;
preferably, the immunoglobulin Fc domain is an Fc domain of IgG, such as an IgG1, IgG2, IgG3 or IgG4 heavy chain constant region;
preferably, the immunoglobulin Fc domain comprises a sequence as set forth in SEQ ID NO: 28 or 29, or a sequence having a substitution, deletion or addition of one or more amino acids or any combination thereof (e.g., a sequence having a substitution, deletion or addition of up to 20, up to 15, up to 10, or up to 5 amino acids or any combination thereof; for example, a sequence having a substitution, deletion or addition of 1, 2, 3, 4 or 5 amino acids or any combination thereof) as compared thereto;
preferably, the polypeptide construct has a sequence as set forth in any one of SEQ ID NOs: 30-47.

**5.** An isolated nucleic acid molecule, which encodes the single-domain antibody or antigen-binding fragment thereof according to any one of claims 1 to 3, or the polypeptide construct according to claim 4.

**6.** A vector, which comprises the nucleic acid molecule according to claim 5; preferably, the vector is a cloning vector or an expression vector.

**7.** A host cell, which comprises the nucleic acid molecule according to claim 5 or the vector according to claim 6.

**8.** A method for preparing the single-domain antibody or antigen-binding fragment thereof according to any one of claims 1 to 3 or the polypeptide construct according to claim 4, which comprises under a condition that allows protein expression, culturing the host cell according to claim 7, and recovering the single-domain antibody or antigen-binding fragment thereof or the polypeptide construct from a culture of the cultured host cell.

**9.** A bispecific or multispecific antibody, which comprises the single-domain antibody or antigen-binding fragment thereof according to any one of claims 1 to 3 or the polypeptide construct according to claim 4;

preferably, the bispecific or multispecific antibody specifically binds to CDCP1 and additionally specifically binds to one or more additional targets;
preferably, the bispecific or multispecific antibody further comprises at least one second antibody having a second binding specificity for a second target.

**10.** A conjugate, which comprises the single-domain antibody or antigen-binding fragment thereof according to any one of claims 1 to 3 or the polypeptide construct according to claim 4, and a therapeutic agent linked to the single-domain antibody or antigen-binding fragment thereof;

preferably, the therapeutic agent is selected from a cytotoxic agent;
preferably, the therapeutic agent is selected from the group consisting of alkylating agent, mitotic inhibitor, antitumor antibiotic, antimetabolite, topoisomerase inhibitor, tyrosine kinase inhibitor, radionuclide agent, and any combination thereof;
preferably, the conjugate is an antibody-drug conjugate (ADC).

**11.** A pharmaceutical composition, which comprises the single-domain antibody or antigen-binding fragment thereof according to any one of claims 1 to 3 or the polypeptide construct according to claim 4, or the bispecific or multispecific antibody according to claim 9 or the conjugate according to claim 10, and a pharmaceutically acceptable carrier and/or excipient;

preferably, the pharmaceutical composition further comprises an additional pharmaceutically active agent;
preferably, the additional pharmaceutically active agent is a drug with anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor or an oncolytic virus;
preferably, the single-domain antibody or antigen-binding fragment thereof, the bispecific or multispecific antibody or the conjugate and the additional pharmaceutically active agent are provided as separate components or as components of the same composition.

**12.** A kit, which comprises the single-domain antibody or antigen-binding fragment thereof according to any one of claims 1 to 3 or the polypeptide construct according to claim 4;

preferably, the single-domain antibody or antigen-binding fragment thereof or the polypeptide construct carries a detectable label, such as an enzyme (e.g., horseradish peroxidase), a radionuclide, a fluorescent dye, a luminescent substance (e.g., a chemiluminescent substance) or biotin;
preferably, the kit further comprises a second antibody, which specifically recognizes the single-domain antibody or antigen-binding fragment thereof according to any one of claims 1 to 3 or the polypeptide construct according to claim 4;
preferably, the second antibody further comprises a detectable label, such as an enzyme (e.g., horseradish peroxidase), a radionuclide, a fluorescent dye, a luminescent substance (e.g., a chemiluminescent substance) or biotin.

**13.** A chimeric antigen receptor, which comprises an antigen-binding domain of the single-domain antibody or antigen-binding fragment thereof according to any one of claims 1 to 3 or the polypeptide construct according to claim 4; preferably, the antigen-binding domain is expressed by an immune effector cell (e.g., a T cell).

**14.** An isolated nucleic acid molecule, which encodes the chimeric antigen receptor according to claim 13.

**15.** A vector, which comprises the isolated nucleic acid molecule according to claim 14; preferably, it is used to prepare a chimeric antigen receptor T cell.

**16.** A host cell, which comprises the isolated nucleic acid molecule according to claim 14 or the vector according to claim 15;

preferably, the host cell is an immune effector cell (e.g., a T cell or a NK cell);
preferably, the host cell is a chimeric antigen receptor T cell (CAR-T).

**17.** Use of the single-domain antibody or antigen-binding fragment thereof according to any one of claims 1 to 3, or the polypeptide construct according to claim 4, or the bispecific or multispecific antibody according to claim 9, or the conjugate according to claim 10, or the pharmaceutical composition according to claim 11, or the kit according to claim 12, or the chimeric antigen receptor according to claim 13, in the manufacture of a medicament for preventing and/or treating a tumor in a subject; preferably, the tumor expresses CDCP1;

preferably, the medicament is used for inhibiting the growth of a tumor cell expressing CDCP1 and/or kill the tumor cell;
preferably, the medicament further comprises an additional pharmaceutically active agent;
preferably, the additional pharmaceutically active agent is a drug with anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor or an oncolytic virus;
preferably, the tumor is selected from the group consisting of non-small cell lung cancer, small cell lung cancer, renal cell carcinoma, colorectal cancer, ovarian cancer, breast cancer, pancreatic cancer, breast cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin lymphoma (CHL), primary mediastinal large B-cell lymphoma, B-cell-rich lymphoma of T-cell/histiocyte, EBV-positive and -negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin lymphoma, central nervous system (CNS) tumors, such as primary CNS lymphoma, spinal axis tumors, brainstem glioma;
preferably, the subject is a mammal, such as a human.

**18.** Use of the single-domain antibody or antigen-binding fragment thereof according to any one of claims 1 to 3 or the polypeptide construct according to claim 4 in the manufacture of a kit for detecting whether a tumor can be treated by an anti-tumor therapy targeting CDCP1;

preferably, the single-domain antibody or antigen-binding fragment thereof carries a detectable label;
preferably, the tumor is selected from the group consisting of non-small cell lung cancer, small cell lung cancer, renal cell carcinoma, colorectal cancer, ovarian cancer, breast cancer, pancreatic cancer, breast cancer, gastric cancer, bladder cancer, esophageal cancer, mesothelioma, melanoma, head and neck cancer, thyroid cancer, sarcoma, prostate cancer, glioblastoma, cervical cancer, thymic carcinoma, leukemia, lymphoma, myeloma, mycosis fungoids, Merkel cell carcinoma and other hematological malignancies, such as classical Hodgkin lymphoma (CHL), primary mediastinal large B-cell lymphoma, B-cell-rich lymphoma of T-cell/histiocyte, EBV-positive and -negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin lymphoma, central nervous system (CNS) tumors, such as primary CNS lymphoma, spinal axis tumors, brainstem gliomas.

**19.** A method for detecting the presence or amount of CDCP1 (e.g., human CDCP1) in a sample, comprising the following steps:

(1) contacting the sample with the single-domain antibody or antigen-binding fragment thereof according to any one of claims 1 to 3 or the polypeptide construct according to claim 4;
(2) detecting the formation of a complex between the single-domain antibody or antigen-binding fragment thereof or the polypeptide construct and CDCP1 or detecting the amount of the complex;

preferably, the single-domain antibody or antigen-binding fragment thereof or the polypeptide construct carries a detectable label.

**20.** A method for reducing an expression level of CDCP1 on the surface of a cell, comprising contacting the cell with the single-domain antibody or antigen-binding fragment thereof according to any one of claims 1 to 3, or the polypeptide construct according to claim 4, or the bispecific or multispecific antibody according to claim 9, or the conjugate according to claim 10, or the pharmaceutical composition according to claim 11, or the kit according to claim 12, so that the expression level of CDCP1 on the surface of the cell is reduced; wherein the cell expresses CDCP1 on its surface; preferably, the cell is a tumor cell expressing CDCP1.

**21.** A method for preventing and/or treating a tumor, the method comprising administering to a subject in need thereof an effective amount of the single-domain antibody or an antigen-binding fragment thereof according to any one of claims 1 to 3, or the polypeptide construct according to claim 4, or the bispecific or multispecific antibody according to claim 9, or the conjugate according to claim 10, or the pharmaceutical composition according to claim 11, or the kit according to claim 12, or the chimeric antigen receptor according to claim 13;

preferably, the tumor expresses CDCP1;
preferably, the tumor is selected from the group consisting of non-small cell lung cancer, small cell lung cancer, renal cell carcinoma, colorectal cancer, ovarian cancer, breast cancer, pancreatic cancer, breast cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin lymphoma (CHL), primary mediastinal large B-cell lymphoma, B-cell-rich lymphoma of T-cell/histiocyte, EBV-positive and -negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin lymphoma, central nervous system (CNS) tumors, such as primary CNS lymphoma, spinal axis tumors, brainstem glioma;
preferably, the subject is a mammal, such as a human.

**22.** A method for detecting whether a tumor can be treated by an anti-tumor therapy targeting CDCP1, the method comprising administering to a subject in need thereof an effective amount of the single-domain antibody or antigen-binding fragment thereof according to any one of claims 1 to 3 or the polypeptide construct according to claim 4;

preferably, the single-domain antibody or antigen-binding fragment thereof carries a detectable label;
preferably, the tumor is selected from the group consisting of non-small cell lung cancer, small cell lung cancer, renal cell carcinoma, colorectal cancer, ovarian cancer, breast cancer, pancreatic cancer, breast cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin lymphoma (CHL), primary mediastinal large B-cell lymphoma, B-cell-rich lymphoma of T-cell/histiocyte, EBV-positive and -negative PTLD and EBV-related diffuse large B-cell lymphoma (DLBCL), plasmablastic lymphoma, extranodal NK/T-cell lymphoma, nasopharyngeal carcinoma and HHV8-related primary effusion lymphoma, Hodgkin lymphoma, central nervous system (CNS) tumors, such as primary CNS lymphoma, spinal axis tumors, brainstem glioma.

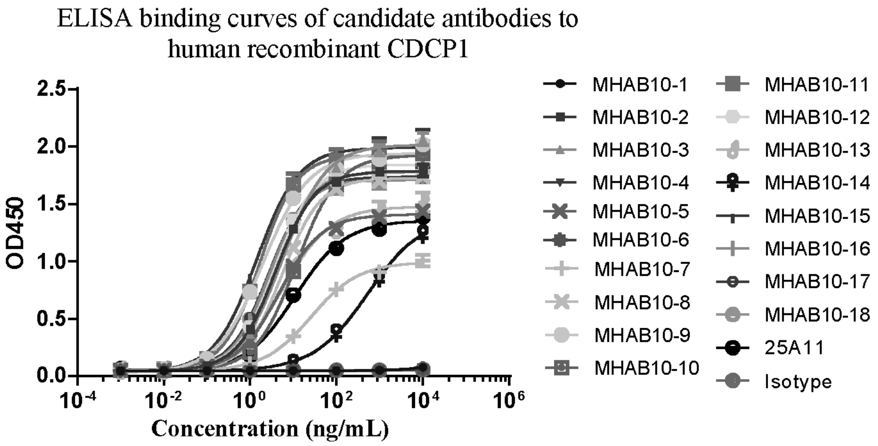
ELISA binding curves of candidate antibodies to human recombinant CDCP1
OD450
2.5
2.0
1.5
1.0
0.5
0.0
$10^{-4}$
$10^{-2}$
$10^{0}$
$10^{2}$
$10^{4}$
$10^{6}$
Concentration (ng/mL)
MHAB10-1
MHAB10-2
MHAB10-3
MHAB10-4
MHAB10-5
MHAB10-6
MHAB10-7
MHAB10-8
MHAB10-9
MHAB10-10
MHAB10-11
MHAB10-12
MHAB10-13
MHAB10-14
MHAB10-15
MHAB10-16
MHAB10-17
MHAB10-18
25A11
Isotype

Fig. 1

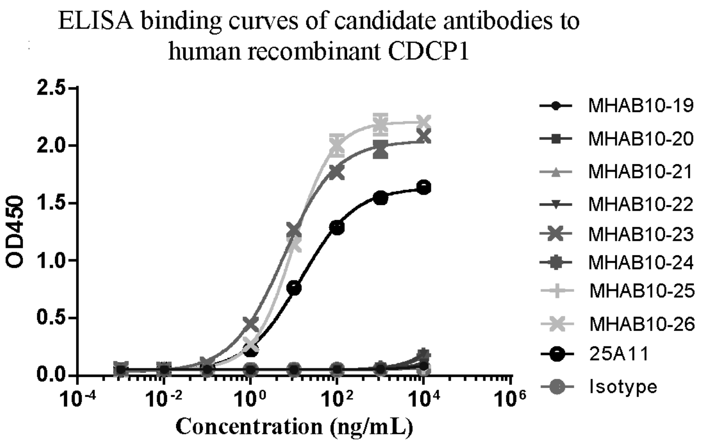
ELISA binding curves of candidate antibodies to human recombinant CDCP1
OD450
2.5
2.0
1.5
1.0
0.5
0.0
$10^{-4}$
$10^{-2}$
$10^{0}$
$10^{2}$
$10^{4}$
$10^{6}$
Concentration (ng/mL)
MHAB10-19
MHAB10-20
MHAB10-21
MHAB10-22
MHAB10-23
MHAB10-24
MHAB10-25
MHAB10-26
25A11
Isotype

Fig. 2

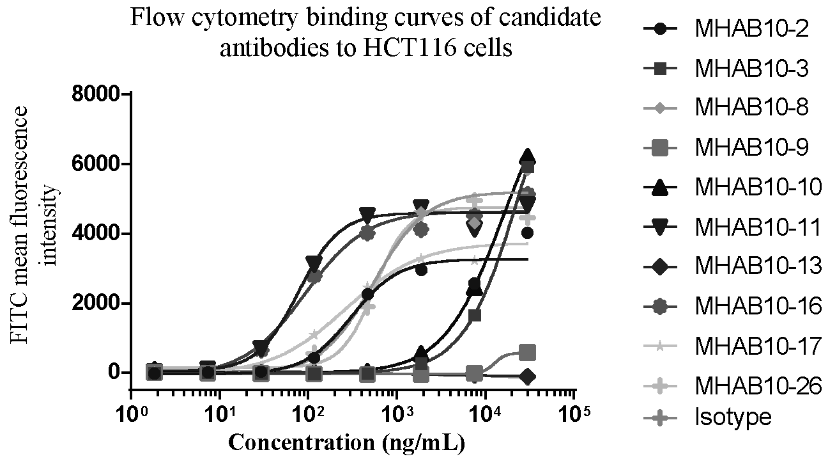


**Fig. 3A**

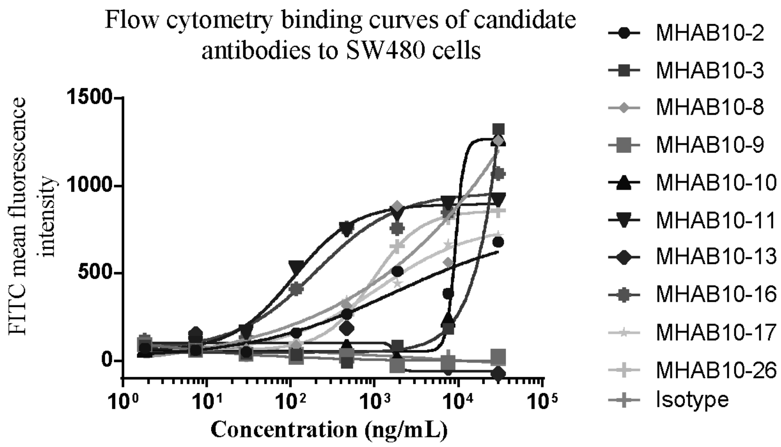


**Fig. 3B**

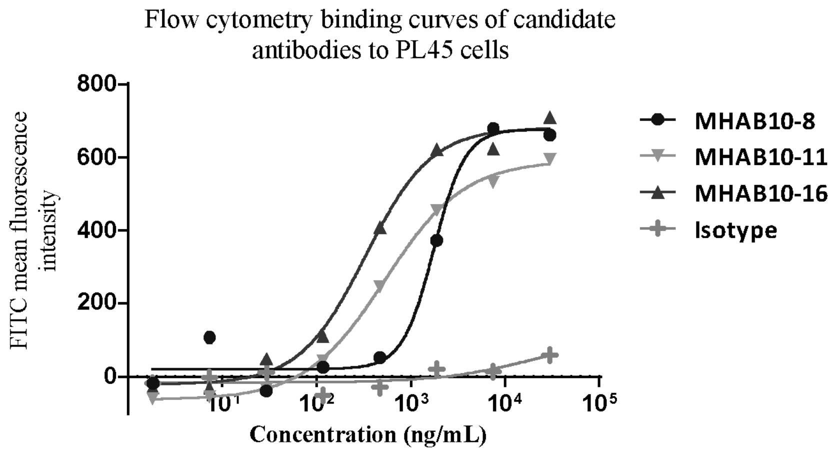


**Fig. 4**

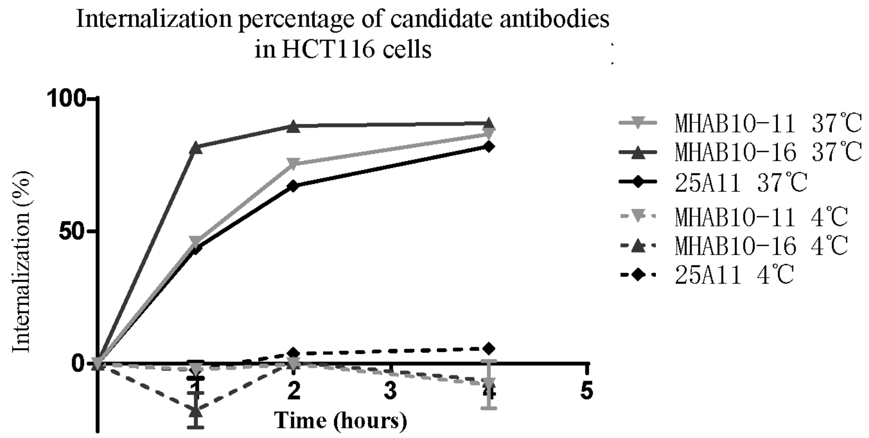
Internalization percentage of candidate antibodies
in HCT116 cells
100
50
0
Internalization (%)
1
2
3
4
5
Time (hours)
MHAB10-11 37℃
MHAB10-16 37℃
25A11 37℃
MHAB10-11 4℃
MHAB10-16 4℃
25A11 4℃

Fig. 5A

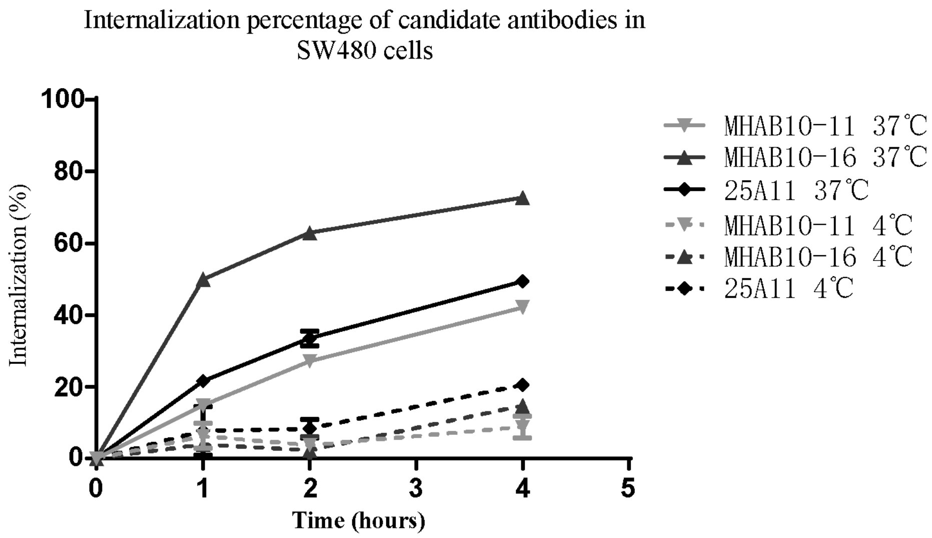
Internalization percentage of candidate antibodies in
SW480 cells
100
80
60
40
20
0
Internalization (%)
0
1
2
3
4
5
Time (hours)
MHAB10-11 37℃
MHAB10-16 37℃
25A11 37℃
MHAB10-11 4℃
MHAB10-16 4℃
25A11 4℃

Fig. 5B

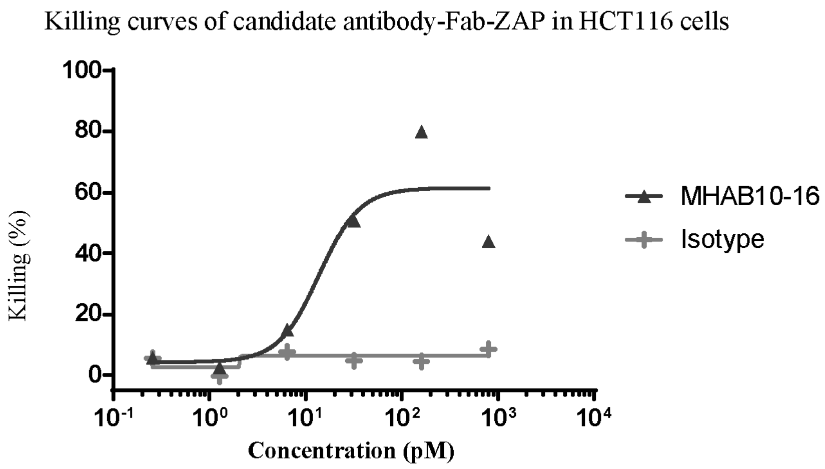


**Fig. 6**

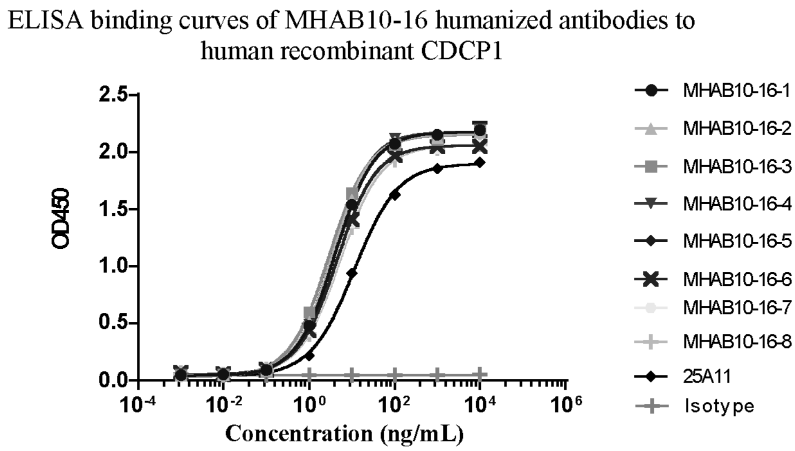


**Fig. 7A**

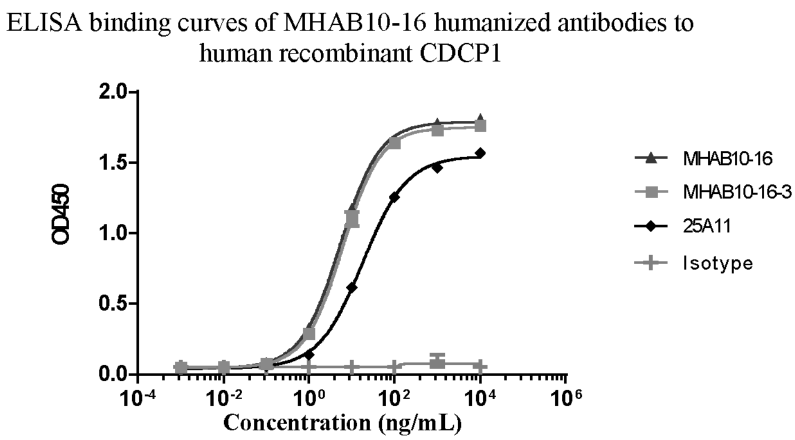


**Fig. 7B**

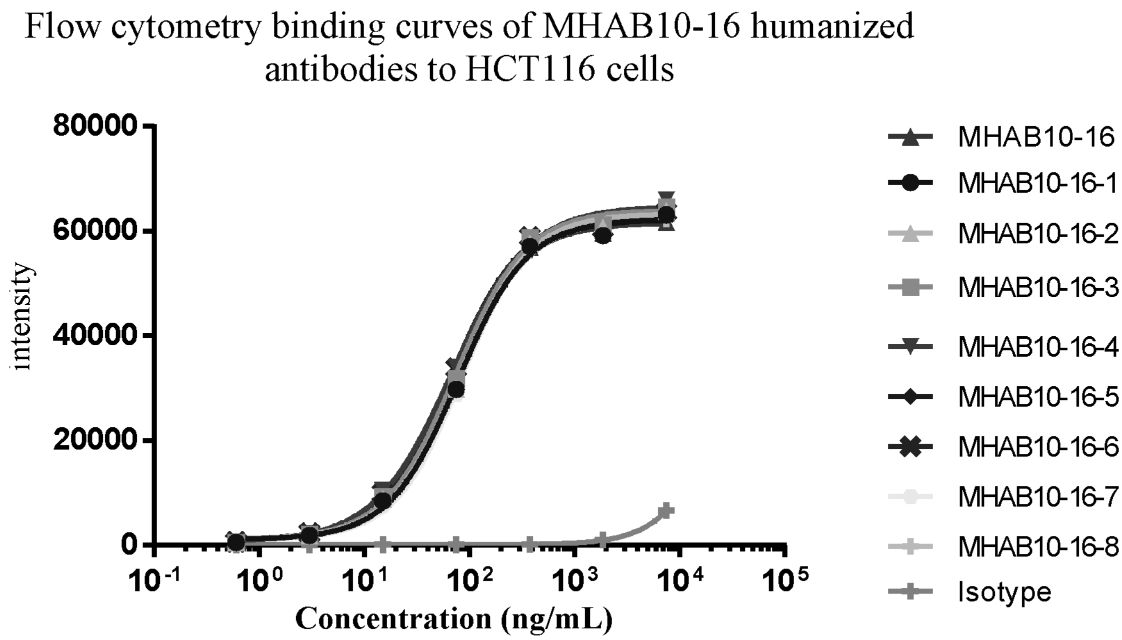
Flow cytometry binding curves of MHAB10-16 humanized antibodies to HCT116 cells
80000
60000
40000
20000
0
intensity
$10^{-1}$
$10^{0}$
$10^{1}$
$10^{2}$
$10^{3}$
$10^{4}$
$10^{5}$
Concentration (ng/mL)
MHAB10-16
MHAB10-16-1
MHAB10-16-2
MHAB10-16-3
MHAB10-16-4
MHAB10-16-5
MHAB10-16-6
MHAB10-16-7
MHAB10-16-8
Isotype

**Fig. 8**

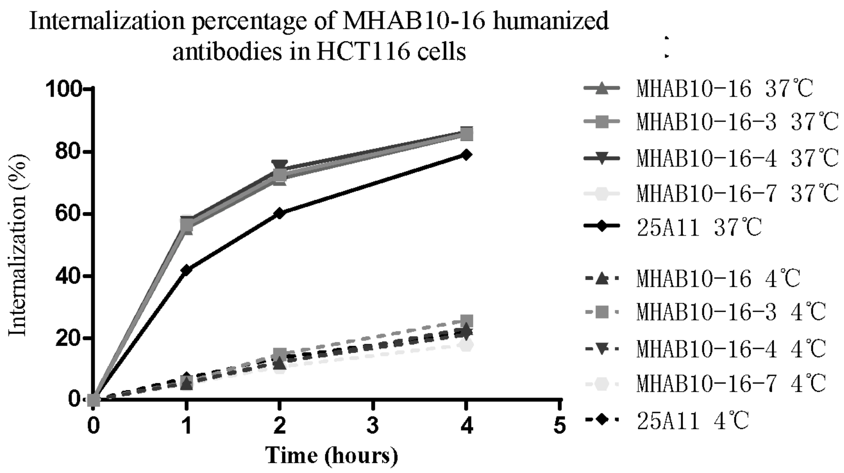
Internalization percentage of MHAB10-16 humanized antibodies in HCT116 cells
100
80
60
40
20
0
Internalization (%)
0
1
2
3
4
5
Time (hours)
MHAB10-16 37℃
MHAB10-16-3 37℃
MHAB10-16-4 37℃
MHAB10-16-7 37℃
25A11 37℃
MHAB10-16 4℃
MHAB10-16-3 4℃
MHAB10-16-4 4℃
MHAB10-16-7 4℃
25A11 4℃

**Fig. 9**

Killing curves of MHAB10-16 humanized antibody-Fab-ZAP in HCT116 cells

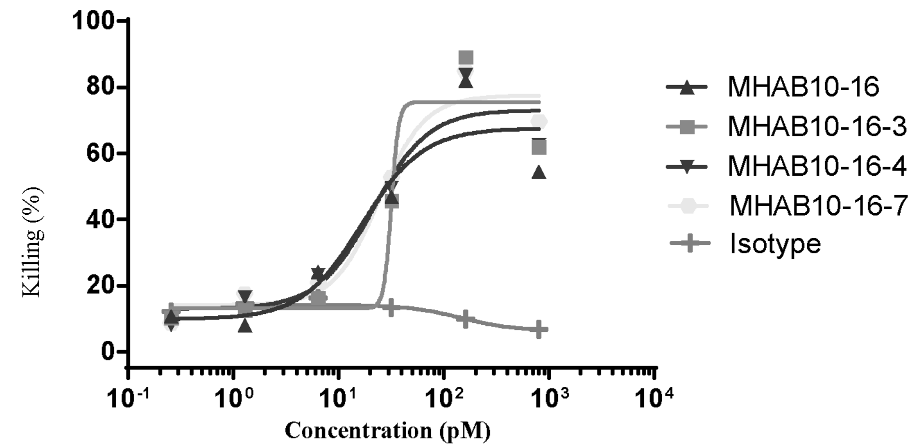


**Fig. 10**

ELISA binding curves of MHAB10-16 humanized antibodies to human recombinant CDCP1

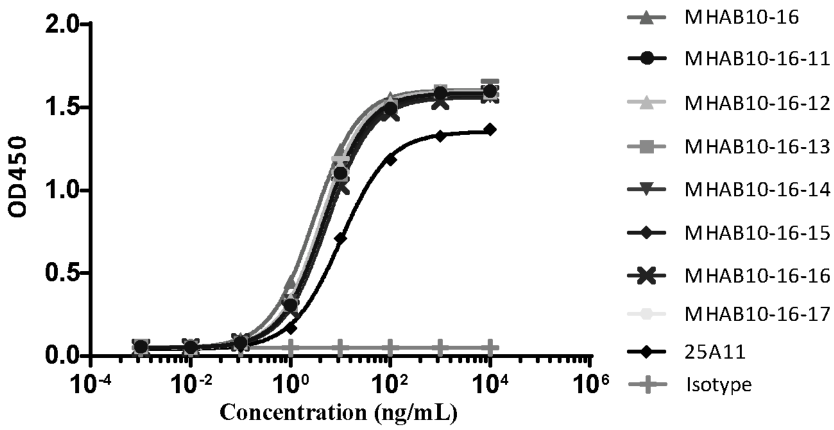


**Fig. 11**

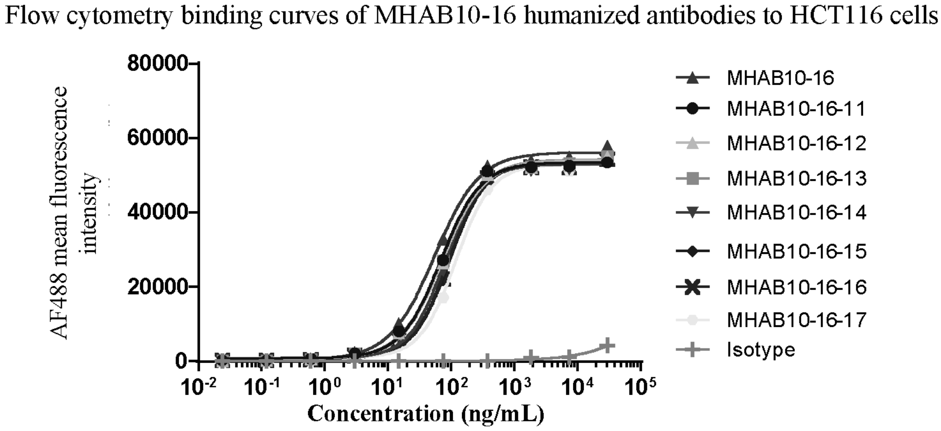


**Fig. 12**

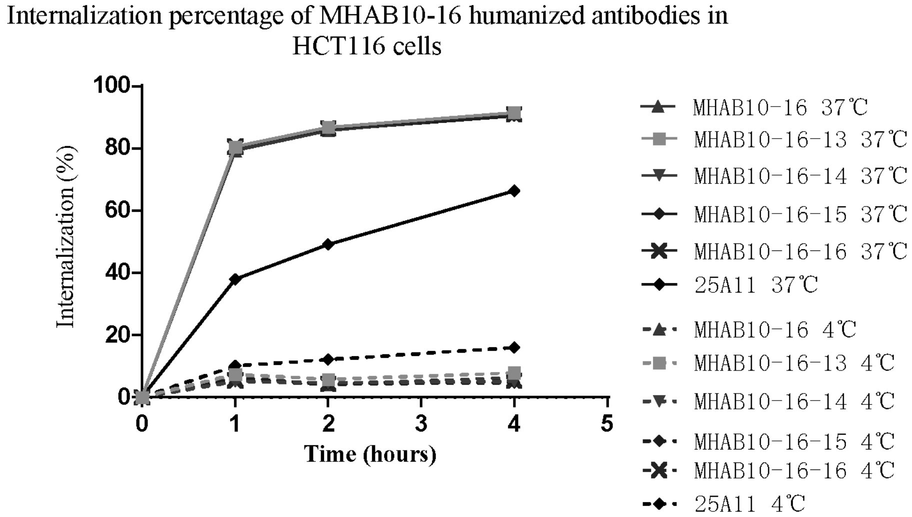


**Fig. 13**

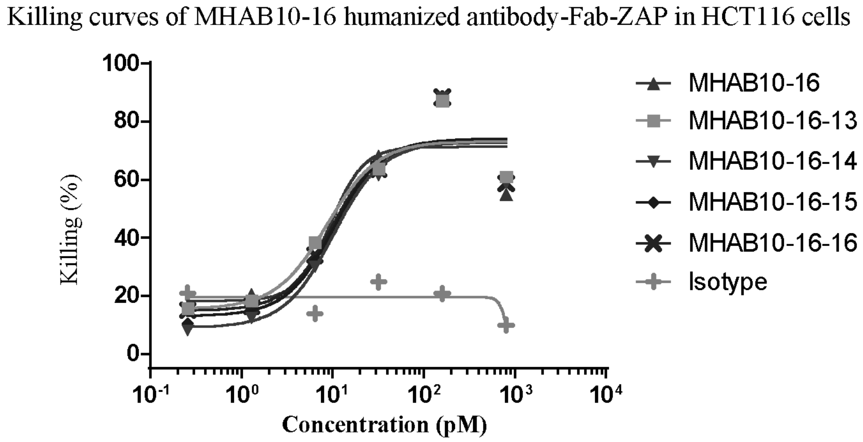


**Fig. 14**

ELISA binding curves of MHAB10-16-14 to CDCP1 antigens of different species

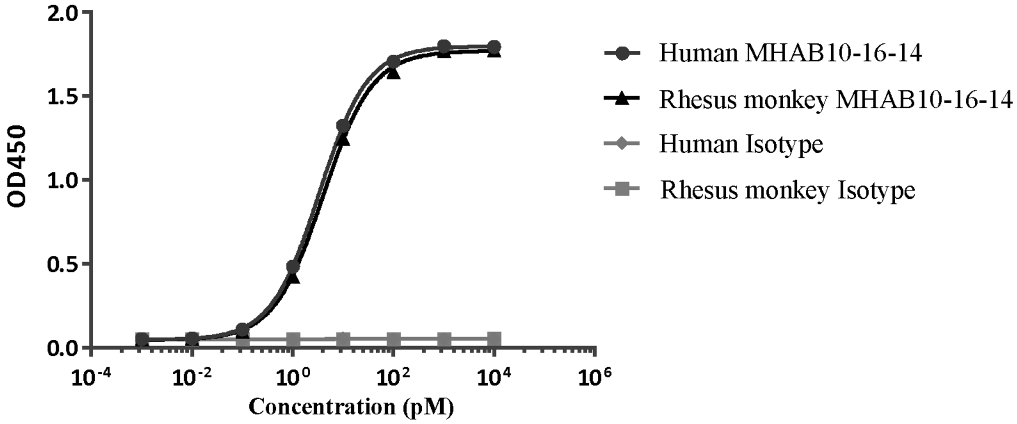


**Fig. 15**

Flow cytometry binding curve of MHAB10-16-14 to HCT116 cells

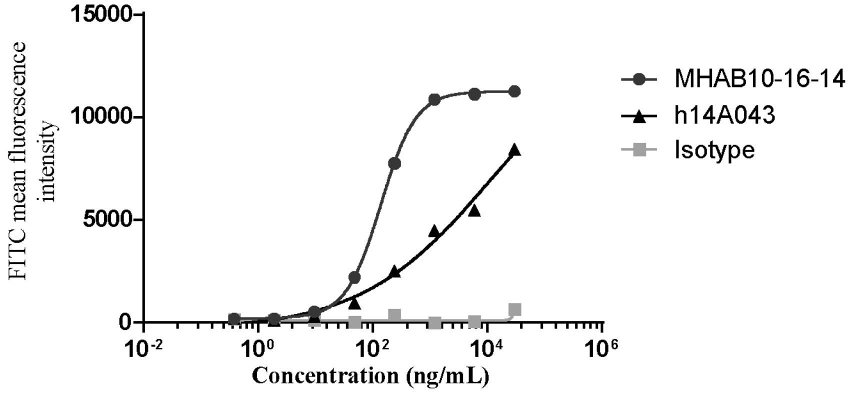


**Fig. 16**

Flow cytometry binding curve of MHAB10-16-14 to SW480 cells

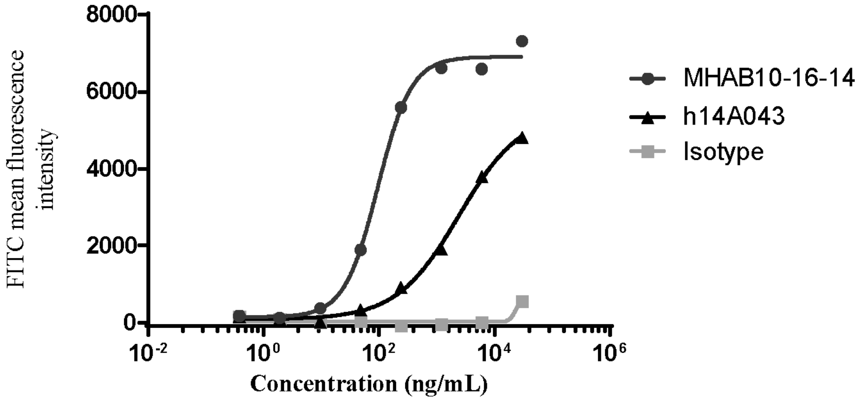


**Fig. 17**

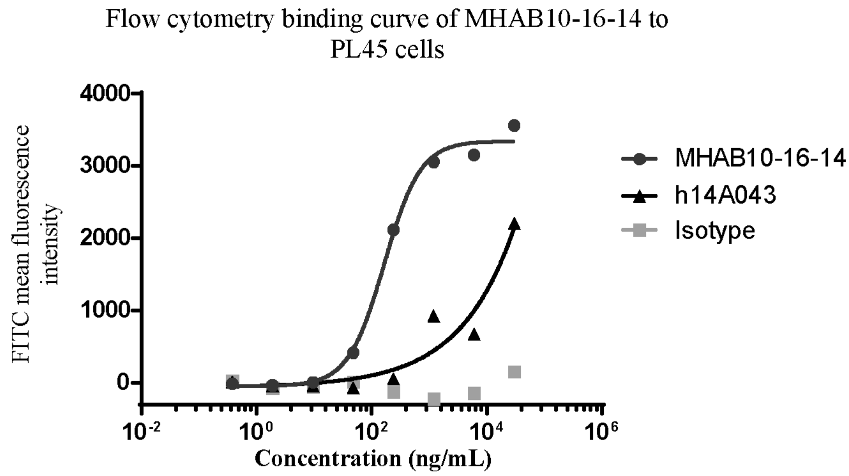


Fig. 18

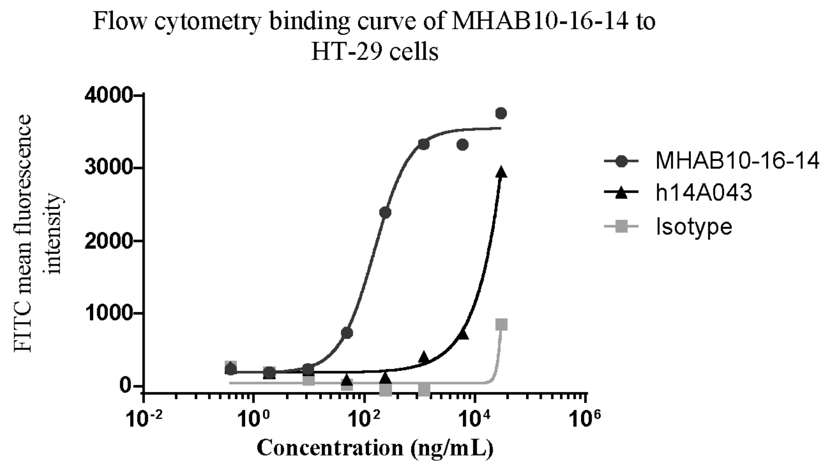


Fig. 19

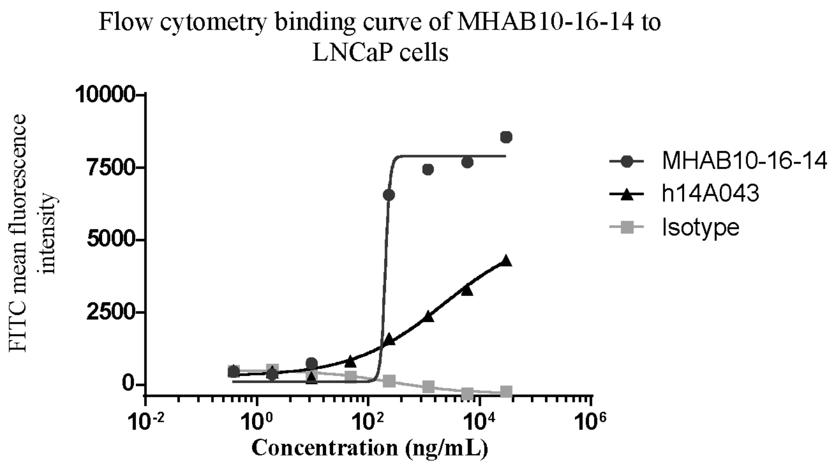


Fig. 20

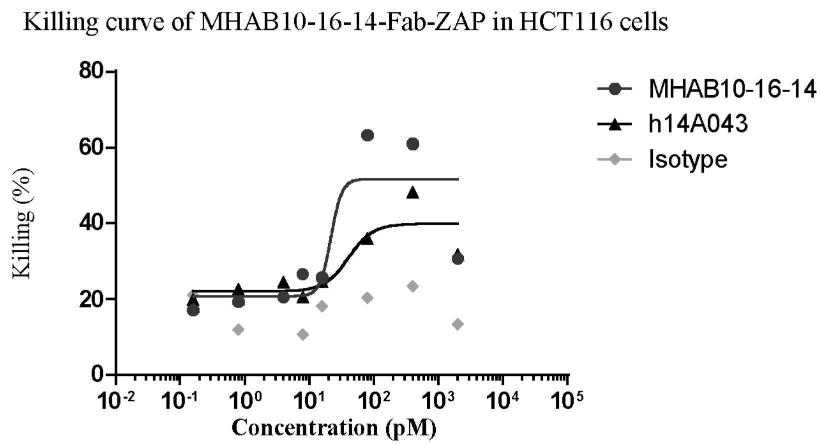
Killing curve of MHAB10-16-14-Fab-ZAP in HCT116 cells
80
60
40
20
0
Killing (%)
10-2 10-1 10⁰ 10¹ 10² 10³ 10⁴ 10⁵
Concentration (pM)
MHAB10-16-14
h14A043
Isotype

Fig. 21

**INTERNATIONAL SEARCH REPORT**

International application No.
**PCT/CN2024/111997**

**A. CLASSIFICATION OF SUBJECT MATTER**

C12N15/13(2006.01)i; A61K39/395(2006.01)i; A61P35/00(2006.01)i; C07K16/28(2006.01)i

According to International Patent Classification (IPC) or to both national classification and IPC

**B. FIELDS SEARCHED**

Minimum documentation searched (classification system followed by classification symbols)

IPC:C12N,C07K,A61K,A61P

Documentation searched other than minimum documentation to the extent that such documents are included in the fields searched

Electronic data base consulted during the international search (name of data base and, where practicable, search terms used)

CNTXT, DWPI, ENTXT, ENTXTC, VEN, WPABS, WPABSC, PUBMED, ISI WEB OF SCIENCE, GenBank, STN: 明慧医药(杭州)有限公司, CUB结构域包含蛋白1, 单域抗体, single-domain antibody, sdAb, 纳米抗体, nanobody, CDCP1, VHH, CDR, cancer, tumor, 肿瘤, 癌症, SEQ ID NOs: 1-27

**C. DOCUMENTS CONSIDERED TO BE RELEVANT**

| Category* | Citation of document, with indication, where appropriate, of the relevant passages | Relevant to claim No. |
|---|---|---|
| A | CN 115176013 A (CHIOME BIOSCIENCE INC.) 11 October 2022 (2022-10-11)<br>claims 1-10 | 1-22 |
| A | US 2010239582 A1 (UCB PHARMA S.A.) 23 September 2010 (2010-09-23)<br>description, paragraphs 8-24 | 1-22 |
| A | WO 2022040506 A2 (YALE UNIVERSITY) 24 February 2022 (2022-02-24)<br>claims 1-24 | 1-22 |
| A | WO 2022206900 A1 (JIANGSU SIMCERE PHARMACEUTICAL CO., LTD.) 06 October 2022 (2022-10-06)<br>claims 1-10 | 1-22 |
| A | WO 2022212876 A1 (THE REGENTS OF UNIVERSITY OF CALIFORNIA) 06 October 2022 (2022-10-06)<br>description, paragraphs 8-30 | 1-22 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **12 November 2024** | **19 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/111997**

**C. DOCUMENTS CONSIDERED TO BE RELEVANT**

| Category* | Citation of document, with indication, where appropriate, of the relevant passages | Relevant to claim No. |
|---|---|---|
| A | MOROZ, A. et al. "Theranostic Targeting of CUB Domain Containing Protein 1 (CDCP1) in Pancreatic Cancer" *Clinical Cancer Research,* Vol. 26, No. (14), 15 July 2020 (2020-07-15), pages 3608-3615 | 1-22 |
| A | 李玲霞 等 (LI, Lingxia et al.). "CDCP1胞外段基因的克隆及在大肠杆菌中表达 (Cloning and Expression of CDCP1 Gene Encoding its Extracellular Domain in Escherichia coli)" *中国实验诊断学 (Chinese Journal of Laboratory Diagnosis),* Vol. 18, No. (7), 31 July 2014 (2014-07-31), pages 1063-1065 | 1-22 |

Form PCT/ISA/210 (second sheet) (July 2022)

**INTERNATIONAL SEARCH REPORT**

International application No.
**PCT/CN2024/111997**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/111997**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **21**
because they relate to subject matter not required to be searched by this Authority, namely:

   Claim 21 relates to a method for preventing and/or treating tumors, which falls within methods for treatment of diseases as defined in PCT Rule 39.1(iv). However, a search is still performed on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/111997**

| Patent document cited in search report | Publication date (day/month/year) | Patent family member(s) | Publication date (day/month/year) |
|---|---|---|---|
| CN 115176013 A | 11 October 2022 | KR 20220119133 A | 26 August 2022 |
| | | US 2023050380 A1 | 16 February 2023 |
| | | EP 4083211 A1 | 02 November 2022 |
| | | EP 4083211 A4 | 03 July 2024 |
| | | TW 202130660 A | 16 August 2021 |
| | | JPWO 2021132427 A1 | 01 July 2021 |
| | | AU 2020413304 A1 | 14 July 2022 |
| | | WO 2021132427 A1 | 01 July 2021 |
| | | IL 294218 A | 01 August 2022 |
| | | CA 3166184 A1 | 01 July 2021 |
| US 2010239582 A1 | 23 September 2010 | EP 2535351 A2 | 19 December 2012 |
| | | EP 2535351 A3 | 03 April 2013 |
| | | US 2019127489 A1 | 02 May 2019 |
| | | US 11427650 B2 | 30 August 2022 |
| | | US 2016311930 A1 | 27 October 2016 |
| | | US 9828438 B2 | 28 November 2017 |
| | | CA 2700714 A1 | 02 April 2009 |
| | | CA 2700714 C | 11 September 2018 |
| | | US 8629246 B2 | 14 January 2014 |
| | | EP 2535349 A1 | 19 December 2012 |
| | | ES 2667729 T3 | 14 May 2018 |
| | | EP 2535350 A1 | 19 December 2012 |
| | | EP 2535350 B1 | 24 January 2018 |
| | | US 2014194596 A1 | 10 July 2014 |
| | | US 9309327 B2 | 12 April 2016 |
| | | WO 2009040562 A1 | 02 April 2009 |
| | | US 2018118853 A1 | 03 May 2018 |
| | | US 10100130 B2 | 16 October 2018 |
| | | ES 2622460 T3 | 06 July 2017 |
| | | EP 2195341 A1 | 16 June 2010 |
| | | EP 2195341 B1 | 22 March 2017 |
| | | JP 2015002744 A | 08 January 2015 |
| | | JP 6106640 B2 | 05 April 2017 |
| | | JP 2010539921 A | 24 December 2010 |
| | | JP 5592792 B2 | 17 September 2014 |
| WO 2022040506 A2 | 24 February 2022 | WO 2022040506 A3 | 07 April 2022 |
| | | US 2023416942 A1 | 28 December 2023 |
| WO 2022206900 A1 | 06 October 2022 | CN 117043184 A | 10 November 2023 |
| WO 2022212876 A1 | 06 October 2022 | EP 4314068 A1 | 07 February 2024 |
| | | JP 2024514530 A | 02 April 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080008719 A1
- US 20200181281 A1
- CN 115176013 A
- US 20230050380 A1


### Non-patent literature cited in the description


- **MORIMOTO et al.** *J. Biochem. Biophys. Methods*, 1992, vol. 24, 107-117
- **BRENNAN et al.** *Science*, 1985, vol. 229, 81
- **HUDSON**. *Curr. Opin. Immunol*, 1999, vol. 11, 548-557
- **LITTLE et al.** *Immunol. Today*, 2000, vol. 21, 364-370
- Fundamental Immunology. Raven Press, 1989
- **KABAT et al.** Sequences of Proteins of Immunological Interest. Public Health Service, 1991
- **CHOTHIA** ; **LESK**. *J. Mol. Biol.*, 1987, vol. 196, 901-917
- **CHOTHIA et al.** *Nature*, 1989, vol. 342, 878-883
- **LEFRANC et al.** *Dev. Comparat. Immunol.*, 2003, vol. 27, 55-77
- **KARLIN** ; **ALTSCHUL**. *Proc. Natl. Acad. Sci. U.S.A.*, 1990, vol. 87, 2264-2268
- **KARLIN** ; **ALTSCHUL**. *Proc. Natl. Acad. Sci. U.S.A.*, 1993, vol. 90, 5873-5877
- **ALTSCHUL et al.** *J. Mol. Biol.*, 1990, vol. 215, 403
- **MALMQVIST M**. *Nature*, 1993, vol. 361, 186-187
- **DAVIES et al.** *Annual Rev Biochem*, 1990, vol. 59, 439-473
- **BRUMMELL et al.** *Biochem.*, 1993, vol. 32, 1180-1187
- **KOBAYASHI et al.** *Protein Eng.*, 1999, vol. 12 (10), 879-884
- **BURKS et al.** *Proc. Natl Acad. Set USA*, 1997, vol. 94, 412-417
- Immunology-A Synthesis. Sinauer Associates, 1991
- **J. SAMBROOK et al.** Molecular cloning: Laboratory Manual. Cold Spring Harbor Laboratory Press, 1989
- **F. M. AUSUBEL et al.** Compiling Guidelines for Molecular Biology Experiments. John Wiley & Sons, Inc, 1995
- **LEFRANC et al.** *Dev. Comparat. Immunol*, 2003, vol. 27, 55-77